# EUROPEAN PATENT APPLICATION

(11) **EP 4 617 269 A1**
(43) Date of publication of application: **17.09.2025**
(21) Application number: 23906231.8
(22) Date of filing: 20.12.2023
(51) Int. Cl.: C07D 401/12, A61K 31/4439, A61K 31/506, A61K 31/501, A61K 31/444, A61K 31/5377, A61K 31/4545, A61P 35/00, C07D 401/14, C07D 403/12

(54) **NOVEL COMPOUND AS RON INHIBITOR**

(30) Priority: 23.12.2022 KR 20220182613
(71) Applicant: LG CHEM, LTD., Seoul 07336 (KR)
(72) Inventor: HAM, Young Jin, Daejeon 34122 (KR); CHO, Joong Heui, Daejeon 34122 (KR); KIM, Jung Joon, Daejeon 34122 (KR); YOON, Hong Bin, Daejeon 34122 (KR); KWAK, Young Shin, Daejeon 34122 (KR); KIM, Woo Sook, Daejeon 34122 (KR); JANG, Sung Woong, Daejeon 34122 (KR)
(74) Representative: Hoffmann Eitle
(86) International application number: PCT/IB2023/062982
(87) International publication number: WO 2024/134517

(57) **Abstract**

The present invention relates to a novel compound as a protein kinase inhibitor, in particular, a RON inhibitor.

## Description

### [Technical Field]

### [Cross-references to Related Application]

This application claims priority to and the benefit of Korean Patent Application No. 10-2022-0182613, filed on December 23, 2022, the disclosure of which is incorporated herein by reference in its entirety.

The present invention relates to a novel compound as a protein kinase inhibitor. Specifically, the present invention relates to a novel compound as a RON inhibitor.

### [Background Art]

At least 400 protein kinases that catalyze a phosphate group transfer reaction from adenosine triphosphate (ATP) to a protein substrate are known. Since the specific amino acid in a target protein to which the phosphate group is transferred is tyrosine, serine, or threonine, protein kinase enzymes are commonly referred to as protein tyrosine kinases (PTKs) or serine/threonine kinases (STKs).

Protein kinases include a large family of structurally related proteins that are required to regulate a wide variety of signaling pathways within cells. The signaling pathway includes many different signaling pathways through the transfer of a phosphate group to target proteins. These phosphorylation processes act as a molecular on/off switch capable of regulating the biological functions of target proteins or protein complexes. The proper function of protein kinases in signaling pathways is to activate or deactivate metabolic enzymes, regulatory proteins, receptors, cytoskeletal proteins, ion channels and pumps, transcription factors, or the like. Improper regulatory signaling due to defective regulation of protein phosphorylation is associated with numerous diseases, including inflammation, cancer, allergies/asthma, diseases of the immune system, diseases of the central nervous system, and angiogenesis.

Most kinases include similar catalytic site consisting of 250 to 300 amino acids. Kinases may be classified according to the substrates they phosphorylate, and sequence motifs that broadly correspond to each of these kinase families have been identified.

Meanwhile, receptor originated from nantes (recepteur d'origine Nantais, RON), one of the tyrosine protein kinase receptors, is also referred to as macrophage stimulating 1 receptor (MST1R), and is reported to promote the invasion and metastasis of cancer cells. It is reported that overexpression of RON also occurs in various tumor types.

Activation of tyrosine protein kinases, such as RON mentioned above, in tumor cells enhances tumor cell proliferation, invasion, and metastasis, and also increases the resistance of tumor cells to cell apoptosis and cytotoxic therapy. Therefore, it is expected that selective small molecule kinase modulators targeting tyrosine protein kinases such as RON have therapeutic potential for the treatment of cancer in which activation of RON receptors or others plays an important role in the development and progression of primary tumors and secondary metastases. Accordingly, continuous research is being conducted on various inhibitors to selectively inhibit the activity of RON, which is one of the tyrosine protein kinases.

### [Disclosure]

### [Technical Problem]

One object of the present invention is to provide a novel compound having protein kinase inhibitory activity.

Another object of the present invention is to provide a compound that is useful for preventing or treating RON-mediated diseases.

Still another object of the present invention is to provide a compound that is useful for preventing or treating cancer or immune diseases.

Yet another object of the present invention is to provide a compound that is useful as a RON inhibitor.

### [Technical Solution]

To achieve the above object,
In one aspect, the present invention provides a compound of the following Chemical Formula 1 or a pharmaceutically acceptable salt thereof.

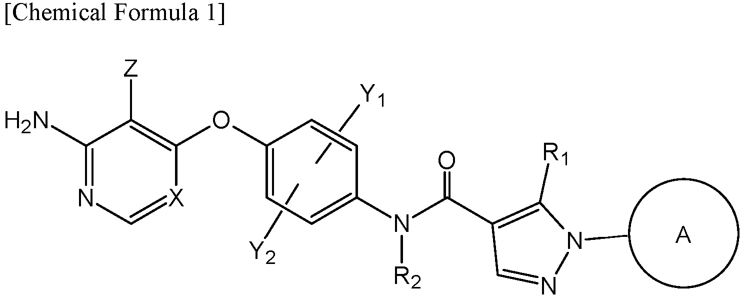

In another aspect, the present invention provides a pharmaceutical composition including: the compound of Chemical Formula 1 or a pharmaceutically acceptable salt thereof; and a pharmaceutically acceptable carrier.

In still another aspect, the present invention provides a pharmaceutical composition for preventing or treating a RON-mediated disease, including the compound of Chemical Formula 1 or a pharmaceutically acceptable salt thereof; and a pharmaceutically acceptable carrier.

In yet another aspect, the present invention provides a pharmaceutical composition for preventing or treating cancer or an immune disease, including the compound of Chemical Formula 1 or a pharmaceutically acceptable salt thereof; and a pharmaceutically acceptable carrier.

### [Advantageous Effects]

According to the present invention, a novel compound or a pharmaceutically acceptable salt thereof that can be effectively used in treating various immune-mediated diseases, for example an anticancer agent, by inhibiting protein kinase activity, especially the protein kinase activity of a RON receptor is provided, but the effect of the present invention is not limited thereto.

### [Best Mode]

First, the terms used in the present invention are defined.

In the present specification, the term "substitution" means that a hydrogen atom bonded to a carbon atom in a structure is changed to another substituent, and a position where substitution occurs is not limited as long as it is a position where the hydrogen atom is substituted, that is, a position where a substituent may be substituted, and when substitution occurs at two or more positions, two or more substituents may be the same or different.

In the present specification, unless otherwise defined, a "substituent" may be deuterium, a halogen, a hydroxy, an alkyl, a cycloalkyl, an alkoxy, an aryloxy, an alkylthioxy, an arylthioxy, an alkylsulfoxy, an arylsulfoxy, an haloalkyl, an alkoxyalkyl, a hydroxyalkyl, an alkenyl, an amine, a nitrile, a nitro, an imide, an amide, an oxo, a carbonyl, a carboxyl, a carbamoyl, an ester, an aryl, a heteroaryl, -(CO)-(CH₂)ₙ-OR_{c} (where n is an integer from 0 to 3, and R_{c} is hydrogen or an alkyl group) or the like.

In the present specification, an "alkyl " is an aliphatic hydrocarbon group that does not include a double bond or triple bond, and it may be a C1 to C20, C1 to C19, C1 to C18, C1 to C17, C1 to C16, C1 to C15, C1 to C14, C1 to C13, C1 to C12, C1 to C11, C1 to C10, for example, C1 to C6, particularly, C1 to C4 alkyl, and it may be a straight chain or a branched chain. Specific examples of the alkyl group include a methyl, an ethyl, a propyl, a n-propyl, an isopropyl, a butyl, a n-butyl, an isobutyl, a tert-butyl, a sec-butyl, a 1-methylbutyl, a 1-ethylbutyl, a pentyl, a n-pentyl, an isopentyl, a neopentyl, a tert-pentyl, a hexyl, n-hexyl, a 1-methylpentyl, a 2-methylpentyl, a 4-methyl-2-pentyl, a 3,3-dimethyl butyl, a 2-ethylbutyl, a heptyl, a n-heptyl, a 1-methylhexyl, an octyl, a n-octyl, a tert-octyl, a 1-methylheptyl, a 2-ethylhexyl, a 2-propylpentyl, a n-nonyl, a 2,2-dimethylheptyl, a 1-ethylpropyl, a 1,1-dimethylpropyl, an isohexyl, a 4-methylhexyl, a 5-methylhexyl, and a benzyl, but are not limited thereto.

In the present specification, a "cycloalkyl " is a cyclic aliphatic hydrocarbon group that does not include a double or triple bond, and it may be a C3 to C30, C3 to C28, C3 to C26, C3 to C24, C3 to C22, C3 to C20, C3 to C18, C3 to C16, C3 to C14, C3 to C12, C3 to C10, for example, C3 to C8, particularly, C3 to C6 cycloalkyl, and it may be monocyclic or polycyclic. The polycyclic group refers to a group in which a cycloalkyl group is directly connected or condensed with another cyclic group, and here, the other cyclic group may be a cycloalkyl, but it may also be another type of cyclic group, for example, a heterocycloalkyl, an aryl, or a heteroaryl. Specific examples of the cycloalkyl group include a cyclopropyl, a cyclobutyl, a cyclopentyl, a 3-methylcyclopentyl, a 2,3-dimethylcyclopentyl, a cyclohexyl, a 3-methylcyclohexyl, a 4-methylcyclohexyl, a 2,3-dimethylcyclohexyl, a 3,4, 5-trimethylcyclohexyl, a 4-tert-butylcyclohexyl, a cycloheptyl, a cyclooctyl, a cyclononyl, a cyclodecyl, a cycloundecyl, a cyclododecyl, a bicyclo[2.2.1]heptyl, a bicyclo[2.2.2]octyl, a bicyclo[3.2.2]nonyl; a bicyclo[4.4.0]decyl; and a bicyclo[4.1.0]heptyl, but are not limited thereto.

In the present specification, a "heterocycloalkyl " is a cyclic aliphatic hydrocarbon group including O, S, Se, N or Si as a heteroatom, and it may be a C2 to C30, C2 to C28, C2 to C26, C2 to C24, C2 to C22, C2 to C20, C2 to C18, C2 to C16, C2 to C14, C2 to C12, C2 to C10, C2 to C8, C2 to C6, for example, C2 to C5 heterocycloalkyl, and it may be monocyclic or polycyclic. The polycyclic group refers to a group in which a heterocycloalkyl group is directly connected or condensed with another cyclic group, and here, the other cyclic group may be a cycloalkyl, but it may also be another type of cyclic group, for example, a heterocycloalkyl, an aryl, or a heteroaryl. Specific examples of the heterocycloalkyl group include aziridine, azetidine, pyrrolidine, piperidine, azepane, azocane, quinuclidine, pyrazolidine, imidazolidine, piperazine (1,2-, 1 , 3- and 1,4-isomers), oxirane, oxetane, tetrahydrofuran, oxane (tetrahydropyran), oxepane, thietane, thiolane (tetrahydrothiophene), thiane (tetrahydrothiopyran), oxazolidine, isoxazolidine, thiazolidine, isothiazolidine, dioxolane, dithiolane, morpholine, thiomorpholine, dioxane, and dithiane, but are not limited thereto.

In the present specification, an "aryl " is an aromatic hydrocarbon group, and it may be a C6 to C30, C6 to C28, C6 to C26, C6 to C24, C6 to C22, C6 to C20, C6 to C18, C6 to C16, C6 to C14, for example, C6 to C12 aryl, and it may be monocyclic or polycyclic. The polycyclic group refers to a group in which an aryl group is directly connected or condensed with another cyclic group, and here, the other cyclic group may be an aryl, but it may also be another type of cyclic group, for example, a cycloalkyl, a heterocycloalkyl, or a heteroaryl. In addition, the aryl group includes a spiro. Specific examples of the aryl group include a phenyl, a biphenyl, a triphenyl, a naphthyl, an anthryl, a chrysenyl, a phenanthrenyl, a perylenyl, a fluoranthenyl, a triphenylenyl, a phenalenyl, a pyrenyl, a tetracenyl, a pentacenyl, a fluorenyl, an indenyl, an acenaphthylenyl, a benzofluorenyl, a spirobifluorenyl, a 2,3-dihydro-1H-indenyl, and condensed cyclic groups thereof, but are not limited thereto.

In the present specification, a "heteroaryl " is an aromatic hydrocarbon group including O, S, Se, N or Si as a heteroatom, and it may be a C3 to C30, C3 to C28, C3 to C26, C3 to C24, C3 to C22, C3 to C20, C3 to C18, C3 to C16, C3 to C14, C3 to C12, C3 to C10, C3 to C8, C3 to C6, for example, C3 to C5 heteroaryl, and it may be monocyclic or polycyclic. The polycyclic group refers to a group in which a heteroaryl group is directly connected or condensed with another cyclic group, and here, the other cyclic group may be a heteroaryl, but it may also be another type of cyclic group, for example, a cycloalkyl, a heterocycloalkyl, an aryl group or the like. Specific examples of the heteroaryl group include a pyridyl, a pyrrolyl, a pyrimidyl, a pyridazinyl, a furanyl, a thiophene, an imidazolyl, a pyrazolyl, an oxazolyl, an isoxazolyl, a thiazolyl, an isothiazolyl, a triazolyl, a furazanyl, an oxadiazolyl, a thiadiazolyl, a dithiazolyl, a tetrazolyl, a pyranyl, a thiopyranyl, a diazinyl, an oxazinyl, a thiazinyl, a dioxinyl, a triazinyl, a tetrazinyl, a quinolyl, an isoquinolyl, a quinazolinyl, an isoquinazolinyl, a quinozolyl, a naphthyridyl, an acridinyl, a phenanthridinyl, an imidazopyridinyl, a diazanaphthalenyl, a triazaindene, an indolyl, an indolizinyl, a benzothiazolyl, a benzoxazolyl, a benzimidazolyl, a benzothiophene, a benzofuran, a dibenzothiophene, a dibenzofuran, a carbazolyl, a benzocarbazolyl, a dibenzocarbazolyl, a phenazinyl, a dibenzosilol, a spirobi (dibenzosilol), dihydrophenazinyl, a phenoxazinyl, a phenanthrenyl, an imidazopyridinyl, a thienyl, an indolo[2,3-a]carbazolyl, an indolo[2,3-b]carbazolyl, an indolinyl, a 10,11-dihydro-dibenzo[b,f]azepine, a 9,10-dihydroacridinyl, a phenanthrazinyl, a phenothiathiazinyl, a phthalazinyl, a naphthylidinyl, a phenanthrolinyl, a benzo[c][1,2,5]thiadiazolyl, a 5,10-dihydrodibenzo[b,e][1,4]azacylinyl, a pyrazolo[1,5-c]quinazolinyl, a pyrido[1,2-b]indazolyl, a pyrido[1,2-a]imidazo[1,2-e]indolinyl, a 5,11-dihydroindeno[1,2-b]carbazolyl. According to one embodiment, the heteroaryl group may be any one selected from the group consisting of a pyridyl, a pyrimidyl, a pyrazolyl, a thiophene, a furanyl, a pyrrolyl, an imidazolyl, a thiazolyl, an oxazolyl, an oxadiazolyl, a triazinyl, a triazolyl, an acridinyl, a pyridazinyl, a pyrazinyl, a quinolyl, a quinazolyl, a quinoxalinyl, an isoquinolyl, an indolyl, an indolinyl, a carbazolyl, a benzoxazolyl, a benzimidazolyl, and a benzothiazolyl, but are not limited thereto.

In the present specification, an "alkoxy " is a group having a chemical structure of -ORa, and Ra may be a substituted or unsubstituted alkyl, wherein the alkyl group is as described above. Specific examples of the alkoxy group include a methoxy, an ethoxy, a n-propoxy, an i-propyloxy, a n-butoxy, an isobutoxy, a tert-butoxy, a sec-butoxy, a n-pentyloxy, a neopentyloxy, an isopentyloxy, n-hexyloxy, a 3,3-dimethyl butyloxy, a 2-ethylbutyloxy, a n-octyloxy, a n-nonyloxy, a n-decyloxy, a benzyloxy, and a p-methylbenzyloxy, but are not limited thereto.

In the present specification, an "amine " is a group having a chemical structure of -NRbRc, and Rb and Rc may each be independently hydrogen, a substituted or unsubstituted alkyl, a substituted or unsubstituted alkenyl, a substituted or unsubstituted alkynyl, a substituted or unsubstituted cycloalkyl, a substituted or unsubstituted heterocycloalkyl, a substituted or unsubstituted aryl, a substituted or unsubstituted heteroaryl, a hydroxyl, and a substituted or unsubstituted alkoxy. The alkyl, the alkenyl, the alkynyl, the cycloalkyl, the heterocycloalkyl, the aryl, the heteroaryl, and the alkoxy group are as described above.

In the present invention, a "pharmaceutically acceptable salt" includes salts commonly used to form alkali metal salts and addition salts of free acids or free bases. The nature of these salts is not important, but they need to be pharmaceutically acceptable. Suitable pharmaceutically acceptable acid addition salts for compounds of Chemical Formula 1 may be prepared from inorganic or organic acids. Examples of these inorganic acids include hydrochloric acid, hydrobromic acid, hydroiodic acid, nitric acid, carbonic acid, sulfuric acid, and phosphoric acid. A suitable organic acid may be selected from aliphatic, alicyclic, aromatic, arylaliphatic, heterocyclic, carboxylic, and sulfonic organic acids, and examples thereof include formic acid, acetic acid, adipic acid, butyric acid, propionic acid, succinic acid, glycol acid, gluconic acid, lactic acid, malic acid, tartaric acid, citric acid, ascorbic acid, glucuronic acid, maleic acid, fumaric acid, pyruvic acid, aspartic acid, glutamic acid, benzoic acid, anthranilic acid, mesylic acid, 4-hydroxybenzoic acid, phenylacetic acid, mandelic acid, embonic acid (pamoic acid), methanesulfonic acid, ethanesulfonic acid, ethanedisulfonic acid, benzenesulfonic acid, pantothenic acid, 2-hydroxyethanesulfonic acid, toluenesulfonic acid, sulfanilic acid, cyclohexylaminosulfonic acid, camphoric acid, camphorsulfonic acid, digluconic acid, cyclopentanepropionic acid, dodecylsulfonic acid, glucoheptanoic acid, glycerophosphonic acid, heptanoic acid, hexanoic acid, 2-hydroxy-ethanesulfonic acid, nicotinic acid, 2-naphthalenesulfonic acid, oxalic acid, palmitic acid, pectic acid, persulfuric acid, 2-phenylpropionic acid, picric acid, pivalic acid, propionic acid, succinic acid, tartaric acid, thiocyanic acid, mesylic acid, undecanoic acid, stearic acid, algenic acid, β-hydroxybutyric acid, salicylic acid, galactaric acid, and galacturonic acid. Pharmaceutically acceptable base addition salts suitable for the compound of Chemical Formula 1 include metal salts, for example, salts prepared from aluminum, calcium, lithium, magnesium, potassium, sodium, and zinc, or salts prepared from organic bases including primary, secondary or tertiary amines, and substituted amines including cyclic amines, for example, caffeine, arginine, diethylamine, N-ethyl piperidine, histidine, glucamine, isopropylamine, lysine, morpholine, N-ethyl morpholine, piperazine, piperidine, triethylamine, and trimethylamine. All of these salts may be prepared from the corresponding compounds of the present invention by conventional methods, such as by reacting the compound of Chemical Formula 1 with a suitable acid or base. When basic and acidic groups are present in a same molecule, the compound of Chemical Formula 1 may also form an internal salt.

In the present invention, the "solvate" may include a hydrate; and a solvate with an organic solvent such as methanol, ethanol, 2-propanol, 1,2-propanediol, 1,3-propanediol, n-butanol, 1,4-butanediol, tert-butanol, acetic acid, acetone, butyl acetate, methyl acetate, ethyl acetate, propyl acetate, t-butyl acetate, isobutyl acetate, methyl ethyl ketone, 2-pentanone, tetrahydrofuran, acetonitrile, chloroform, toluene, and mixtures thereof.

In the present specification, the term "prevention" refers to reduction in the risk of a disease or disorder and means all actions of suppressing or delaying the onset of a disease by preventing one or more clinical symptoms of a disease from progressing in an individual that is exposed to the disease or susceptible to developing the disease but that has not developed the disease or exhibited a symptom of the disease.

In the present specification, the term "treatment" refers to mitigating a disease or disorder and means all actions of ameliorating or beneficially changing symptoms of a disease by arresting or reducing the progression of the disease or one or more clinical symptoms thereof.

In the present specification, the term "carrier" refers to a compound that facilitates the introduction of a compound into cells or tissue. The pharmaceutical composition may be prepared in the form of a unit dose by formulating it using a pharmaceutically acceptable carrier, or may be prepared by placing it into a multi-dose container. At this time, the dosage form may be in the form of a solution, suspension, or emulsion in an oil or aqueous medium, or may be in the form of an extract, powder, granules, tablets, capsules, or gel (e.g., hydrogel), and may further include a dispersant or stabilizer.

In the present specification, the term "therapeutically effective amount" refers to the amount of each formulation that achieves the purpose of improving disease severity and incidence during treatment with each formulation itself, but avoids harmful side effects commonly associated with other treatment methods. For example, an effective tumor treatment agent extends the patient's survival period, inhibits the growth of rapidly proliferating cells associated with the tumor, or is effective in tumor regression.

Hereinafter, the present invention will be described in detail.

### 1. Novel compound as receptor originated from nantes (RON) inhibitor

One aspect of the present invention provides a compound having the chemical structure of Chemical Formula 1 below, or a pharmaceutically acceptable salt, isomer, solvate, or prodrug thereof.

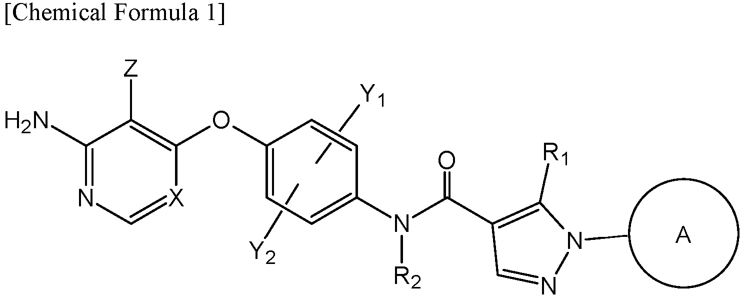

In Chemical Formula 1, X may be N or C.

In Chemical Formula 1, R1 may be any one selected from the group consisting of hydrogen, a halogen, a substituted or unsubstituted alkyl, and a substituted or unsubstituted cycloalkyl, in particular, it may be a halogen, an alkyl, or a haloalkyl, and in particular, it may be an alkyl group or a haloalkyl, for example, a trifluoromethyl group or an ethyl.

In Chemical Formula 1, R2 may be any one selected from the group consisting of hydrogen and a substituted or unsubstituted alkyl, and in particular, it may be hydrogen or an alkoxyalkyl, for example, hydrogen or a methoxypropyl.

In Chemical Formula 1, Y1 and Y2 may each be independently any one selected from the group consisting of hydrogen, a halogen, a substituted or unsubstituted alkyl, and a substituted or unsubstituted cycloalkyl, and in particular, it may each be independently hydrogen or a halogen, and in particular, it may each be independently a halogen, for example, fluorine. In addition, Y1 and Y2 may be connected to carbons Nos. 3 and 5, carbons Nos. 2 and 5, or carbons Nos. 2 and 6 of a benzene ring.

In Chemical Formula 1, Z may be any one selected from the group consisting of hydrogen, a halogen, a substituted or unsubstituted alkyl, a substituted or unsubstituted alkenyl, a substituted or unsubstituted alkynyl, a substituted or unsubstituted cycloalkyl, a substituted or unsubstituted heterocycloalkyl, a substituted or unsubstituted aryl, a substituted or unsubstituted heteroaryl, a nitro, a cyano, and -(CH₂)ₙ-W, in particular, it may be any one selected from the group consisting of hydrogen, a halogen, a substituted or unsubstituted alkyl, a substituted or unsubstituted alkynyl, a substituted or unsubstituted cycloalkyl, a substituted or unsubstituted heterocycloalkyl, a substituted or unsubstituted aryl, a nitro, a cyano, and -(CH₂)ₙ-W, in particular, it may be any one selected from the group consisting of a halogen, a substituted or unsubstituted alkyl, a substituted or unsubstituted alkynyl, a substituted or unsubstituted cycloalkyl, a substituted or unsubstituted heterocycloalkyl, a cyano, and -(CH₂)ₙ-W, and in particular, it may be any one selected from the group consisting of a halogen, an alkyl, an alkynyl, a hydroxyalkynyl, a hydroxyalkylalkynyl, a cycloalkyl, tetrahydropyran, a cyano, and -(CH₂)ₙ-W.

Here, n may be an integer from 0 to 3, in particular, n may be an integer from 1 to 3, in particular, n may be an integer from 1 to 2, and in particular, n may be 1.

In addition, W may be -NRaRb, -ORa or a heterocycloalkyl, in particular, it may be a heterocycloalkyl, and in particular, it may be morpholine, and Ra and Rb may each be independently hydrogen or a substituted or unsubstituted alkyl, and in particular, Ra and Rb may be an alkyl.

In Chemical Formula 1, A may be any one selected from the group consisting of hydrogen, a substituted or unsubstituted alkyl, a substituted or unsubstituted cycloalkyl, a substituted or unsubstituted heterocycloalkyl, a substituted or unsubstituted aryl, and a substituted and unsubstituted heteroaryl, in particular, it may be any one selected from the group consisting of a substituted or unsubstituted alkyl, a substituted or unsubstituted heterocycloalkyl, a substituted or unsubstituted aryl, and a substituted and unsubstituted heteroaryl, in particular, it may be any one selected from the group consisting of a substituted alkyl, a substituted heterocycloalkyl, a substituted or unsubstituted aryl, and a substituted or unsubstituted heteroaryl, and for example, it may be any one selected from the group consisting of an alkoxyalkyl, an aryl, a pyridine, a halopyridine, a pyrimidine, a pyridazine, a piperidine, an alkoxyalkylpiperidine, and a piperidine group substituted with -(CO)-(CH₂)ₙ-ORc, and here n may be 1, and Rc may be an alkyl.

In addition, the compound of Chemical Formula 1 may be selected from the group consisting of:
N-(4-((2-amino-3-chloropyridin-4-yl)oxy)-3,5-difluorophenyl)-1-phenyl-5-(trifluoromethyl)-1H-pyrazole-4-carboxamide;
N-(4-((2-amino-3-chloropyridin-4-yl)oxy)-3,5-difluorophenyl)-1-(pyrimidin-2-yl)-5-(trifluoromethyl)-1H-pyrazole-4-carboxamide;
N-(4-((2-amino-3-chloropyridin-4-yl)oxy)-3,5-difluorophenyl)-1-(pyridazin-3-yl)-5-(trifluoromethyl)-1H-pyrazole-4-carboxamide;
N-(4-((2-amino-3-chloropyridin-4-yl)oxy)-3,5-difluorophenyl)-5-ethyl-1-(3-fluoropyridin-2-yl)-1H-pyrazole-4-carboxamide;
N-(4-((2-amino-3-chloropyridin-4-yl)oxy)-2,5-difluorophenyl)-1-(3-fluoropyridin-2-yl)-5-(trifluoromethyl)-1H-pyrazole-4-carboxamide;
N-(4-((2-amino-3-chloropyridin-4-yl)oxy)-2,5-difluorophenyl)-1-phenyl-5-(trifluoromethyl)-1H-pyrazole-4-carboxamide;
N-(4-((2-amino-3-chloropyridin-4-yl)oxy)-2,6-difluorophenyl)-1-(3-fluoropyridin-2-yl)-5-(trifluoromethyl)-1H-pyrazole-4-carboxamide;
N-(4-((2-amino-3-chloropyridin-4-yl)oxy)-2,6-difluorophenyl)-1-phenyl-5-(trifluoromethyl)-1H-pyrazole-4-carboxamide;
N-(4-((6-amino-5-chloropyrimidin-4-yl)oxy)-3,5-difluorophenyl)-1-phenyl-5-(trifluoromethyl)-1H-pyrazole-4-carboxamide;
N-(4-((6-amino-5-chloropyrimidin-4-yl)oxy)-3,5-difluorophenyl)-1-(3-fluoropyridin-2-yl)-5-(trifluoromethyl)-1H-pyrazole-4-carboxamide;
N-(4-((6-amino-5-chloropyrimidin-4-yl)oxy)-3,5-difluorophenyl)-1-(3-chloropyridin-2-yl)-5-(trifluoromethyl)-1H-pyrazole-4-carboxamide;
N-(4-((6-amino-5-chloropyrimidin-4-yl)oxy)-3,5-difluorophenyl)-1-(pyrimidin-2-yl)-5-(trifluoromethyl)-1H-pyrazole-4-carboxamide;
N-(4-((2-amino-3-iodopyridin-4-yl)oxy)-3,5-difluorophenyl)-1-phenyl-5-(trifluoromethyl)-1H-pyrazole-4-carboxamide;
N-(4-((2-amino-3-iodopyridin-4-yl)oxy)-3,5-difluorophenyl)-1-(pyridin-2-yl)-5-(trifluoromethyl)-1H-pyrazole-4-carboxamide;
N-(4-((2-amino-3-iodopyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-iodopyridin-4-yl)oxy)-3,5-difluorophenyl)-1-(3-chloropyridin-2-yl)-5-(trifluoromethyl)-1H-pyrazole-4-carboxamide;
N-(4-((2-amino-3-iodopyridin-4-yl)oxy)-3,5-difluorophenyl)-1-(pyridazin-3-yl)-5-(trifluoromethyl)-1H-pyrazole-4-carboxamide;
N-(4-((2-amino-3-iodopyridin-4-yl)oxy)-3,5-difluorophenyl)-1-(pyrimidin-2-yl)-5-(trifluoromethyl)-1H-pyrazole-4-carboxamide;
N-(4-((2-amino-3-(tetrahydro-2H-pyran-4-yl)pyridin-4-yl)oxy)-3,5-difluorophenyl)-1-phenyl-5-(trifluoromethyl)-1H-pyrazole-4-carboxamide;
N-(4-((2-amino-3-(tetrahydro-2H-pyran-4-yl)pyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-(tetrahydro-2H-pyran-4-yl)pyridin-4-yl)oxy)-3,5-difluorophenyl)-1-(3-chloropyridin-2-yl)-5-(trifluoromethyl)-1H-pyrazole-4-carboxamide;
N-(4-((2-amino-3-(tetrahydro-2H-pyran-4-yl)pyridin-4-yl)oxy)-3,5-difluorophenyl)-1-(pyrimidin-2-yl)-5-(trifluoromethyl)-1H-pyrazole-4-carboxamide;
N-(4-((2-amino-3-(tetrahydro-2H-pyran-4-yl)pyridin-4-yl)oxy)-3,5-difluorophenyl)-1-(pyridazin-3-yl)-5-(trifluoromethyl)-1H-pyrazole-4-carboxamide;
N-(4-((2-amino-3-(tetrahydro-2H-pyran-4-yl)pyridin-4-yl)oxy)-3,5-difluorophenyl)-1-(pyridin-2-yl)-5-(trifluoromethyl)-1H-pyrazole-4-carboxamide;
N-(4-((2-amino-3-ethynylpyridin-4-yl)oxy)-3,5-difluorophenyl)-1-phenyl-5-(trifluoromethyl)-1H-pyrazole-4-carboxamide;
N-(4-((2-amino-3-ethynylpyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-ethynylpyridin-4-yl)oxy)-3,5-difluorophenyl)-1-(3-chloropyridin-2-yl)-5-(trifluoromethyl)-1H-pyrazole-4-carboxamide;
N-(4-((2-amino-3-ethynylpyridin-4-yl)oxy)-3,5-difluorophenyl)-1-(pyrimidin-2-yl)-5-(trifluoromethyl)-1H-pyrazole-4-carboxamide;
N-(4-((2-amino-3-ethynylpyridin-4-yl)oxy)-3,5-difluorophenyl)-1-(pyridin-2-yl)-5-(trifluoromethyl)-1H-pyrazole-4-carboxamide;
N-(4-((2-amino-3-ethynylpyridin-4-yl)oxy)-3,5-difluorophenyl)-1-(pyridazin-3-yl)-5-(trifluoromethyl)-1H-pyrazole-4-carboxamide;
N-(4-((2-amino-3-cyanopyridin-4-yl)oxy)-3,5-difluorophenyl)-1-phenyl-5-(trifluoromethyl)-1H-pyrazole-4-carboxamide;
N-(4-((2-amino-3-cyanopyridin-4-yl)oxy)-3,5-difluorophenyl)-1-(pyridin-2-yl)-5-(trifluoromethyl)-1H-pyrazole-4-carboxamide;
N-(4-((2-amino-3-cyanopyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-cyanopyridin-4-yl)oxy)-3,5-difluorophenyl)-1-(3-chloropyridin-2-yl)-5-(trifluoromethyl)-1H-pyrazole-4-carboxamide;
N-(4-((2-amino-3-cyanopyridin-4-yl)oxy)-3,5-difluorophenyl)-1-(pyrimidin-2-yl)-5-(trifluoromethyl)-1H-pyrazole-4-carboxamide;
N-(4-((2-amino-3-cyanopyridin-4-yl)oxy)-3,5-difluorophenyl)-1-(pyridazin-3-yl)-5-(trifluoromethyl)-1H-pyrazole-4-carboxamide;
N-(4-((2-amino-3-cyclopropylpyridin-4-yl)oxy)-3,5-difluorophenyl)-1-phenyl-5-(trifluoromethyl)-1H-pyrazole-4-carboxamide;
N-(4-((2-amino-3-cyclopropylpyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-cyclopropylpyridin-4-yl)oxy)-3,5-difluorophenyl)-1-(3-chloropyridin-2-yl)-5-(trifluoromethyl)-1H-pyrazole-4-carboxamide;
N-(4-((2-amino-3-cyclopropylpyridin-4-yl)oxy)-3,5-difluorophenyl)-1-(pyridin-2-yl)-5-(trifluoromethyl)-1H-pyrazole-4-carboxamide;
N-(4-((2-amino-3-cyclopropylpyridin-4-yl)oxy)-3,5-difluorophenyl)-1-(pyrimidin-2-yl)-5-(trifluoromethyl)-1H-pyrazole-4-carboxamide;
N-(4-((2-amino-3-cyclopropylpyridin-4-yl)oxy)-3,5-difluorophenyl)-1-(pyridazin-3-yl)-5-(trifluoromethyl)-1H-pyrazole-4-carboxamide;
N-(4-((2-amino-3-isopropylpyridin-4-yl)oxy)-3,5-difluorophenyl)-1-phenyl-5-(trifluoromethyl)-1H-pyrazole-4-carboxamide;
N-(4-((2-amino-3-isopropylpyridin-4-yl)oxy)-3,5-difluorophenyl)-1-(3-chloropyridin-2-yl)-5-(trifluoromethyl)-1H-pyrazole-4-carboxamide;
N-(4-((2-amino-3-isopropylpyridin-4-yl)oxy)-3,5-difluorophenyl)-1-(pyrimidin-2-yl)-5-(trifluoromethyl)-1H-pyrazole-4-carboxamide;
N-(4-((2-amino-3-isopropylpyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-(morpholinomethyl)pyridin-4-yl)oxy)-3,5-difluorophenyl)-1-(3-fluoropyridine-2-yl)-5-(trifluoromethyl)-1H-pyrazole-4-carboxamide;
N-(4-((2-amino-3-(morpholinomethyl)pyridin-4-yl)oxy)-3,5-difluorophenyl)-1-(pyrimidin-2-yl)-5-(trifluoromethyl)-1H-pyrazole-4-carboxamide;
N-(4-((2-amino-3-(morpholinomethyl)pyridin-4-yl)oxy)-3,5-difluorophenyl)-1-(3-chloropyridin-2-yl)-5-(trifluoromethyl)-1H-pyrazole-4-carboxamide;
N-(4-((2-amino-3-(morpholinomethyl)pyridin-4-yl)oxy)-3,5-difluorophenyl)-1-phenyl-5-(trifluoromethyl)-1H-pyrazole-4-carboxamide;
N-(4-((2-amino-3-(3-hydroxyprop-1-yn-1-yl)pyridin-4-yl)oxy)-3,5-difluorophenyl)-1-phenyl-5-(trifluoromethyl)-1H-pyrazole-4-carboxamide;
N-(4-((2-amino-3-(3-hydroxyprop-1-yn-1-yl)pyridin-4-yl)oxy)-3,5-difluorophenyl)-1-(pyridin-2-yl)-5-(trifluoromethyl)-1H-pyrazole-4-carboxamide;
N-(4-((2-amino-3-(3-hydroxyprop-1-yn-1-yl)pyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-(3-hydroxyprop-1-yn-1-yl)pyridin-4-yl)oxy)-3,5-difluorophenyl)-1-(3-chloropyridin-2-yl)-5-(trifluoromethyl)-1H-pyrazole-4-carboxamide;
N-(4-((2-amino-3-(3-hydroxyprop-1-yn-1-yl)pyridin-4-yl)oxy)-3,5-difluorophenyl)-1-(pyrimidin-2-yl)-5-(trifluoromethyl)-1H-pyrazole-4-carboxamide;
N-(4-((2-amino-3-(3-hydroxyprop-1-yn-1-yl)pyridin-4-yl)oxy)-3,5-difluorophenyl)-1-(pyridazin-3-yl)-5-(trifluoromethyl)-1H-pyrazole-4-carboxamide;
N-(4-((2-amino-3-(3-hydroxybut-1-yn-1-yl)pyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-(3-hydroxy-3-methylbut-1-yn-1-yl)pyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-chloropyridin-4-yl)oxy)-3,5-difluorophenyl)-1-(1-(3-methoxypropyl)piperi din-4-yl)-5-(trifluoromethyl)-1H-pyrazole-4-carboxamide;
N-(4-((2-amino-3-chloropyridin-4-yl)oxy)-3,5-difluorophenyl)-1-(1-(2-ethoxyacetyl)piperi din-4-yl)-5-(trifluoromethyl)-1H-pyrazole-4-carboxamide; and
N-[4-[(2-amino-3-chloro-4-pyridyl)oxy]-3,5-difluoro-phenyl]-1-(3-methoxypropyl)-5-(trifluoromethyl)pyrazole-4-carboxamide, and the structures thereof are as shown in Table 1 below.

**[Table 1]**

| | Structure | Name |
|---|---|---|
| Example 1 | 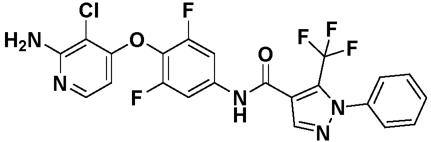 | N-(4-((2-amino-3-chloropyridin-4-yl)oxy)-3,5-difluorophenyl)-1-phenyl-5-(trifluoromethyl)-1H-pyrazole-4-carboxamide |
| Example 2 | 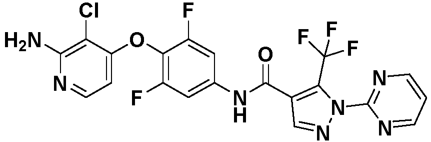 | N-(4-((2-amino-3-chloropyridin-4-yl)oxy)-3,5-difluorophenyl)-1-(pyrimidin-2-yl)-5-(trifluoromethyl)-1H-pyrazole-4-carboxamide |
| Example 3 | 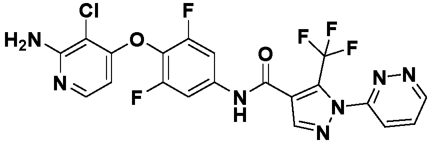 | N-(4-((2-amino-3-chloropyridin-4-yl)oxy)-3,5-difluorophenyl)-1-(pyridazin-3-yl)-5-(trifluoromethyl)-1H-pyrazole-4-carboxamide |
| Example 4 | 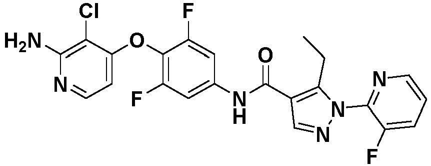 | N-(4-((2-amino-3-chloropyridin-4-yl)oxy)-3,5-difluorophenyl)-5-ethyl-1-(3-fluoropyridin-2-yl)-1H-pyrazole-4-carboxamide |
| Example 5 | 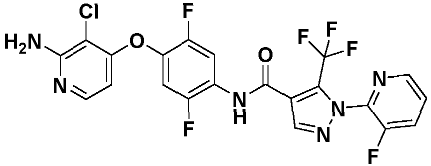 | N-(4-((2-amino-3-chloropyridin-4-yl)oxy)-2,5-difluorophenyl)-1-(3-fluoropyridin-2-yl)-5-(trifluoromethyl)-1H-pyrazole-4-carboxamide |
| Example 6 | 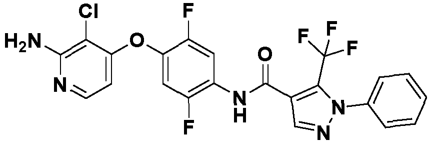 | N-(4-((2-amino-3-chloropyridin-4-yl)oxy)-2,5-difluorophenyl)-1-phenyl-5-(trifluoromethyl)-1H-pyrazole-4-carboxamide |
| Example 7 | 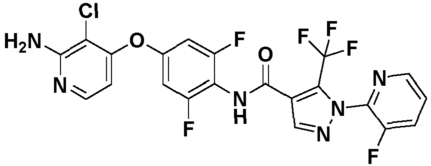 | N-(4-((2-amino-3-chloropyridin-4-yl)oxy)-2,6-difluorophenyl)-1-(3-fluoropyridin-2-yl)-5-(trifluoromethyl)-1H-pyrazole-4-carboxamide |
| Example 8 | 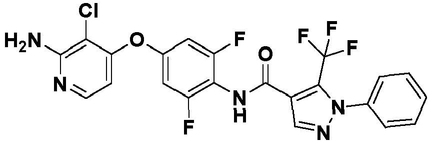 | N-(4-((2-amino-3-chloropyridin-4-yl)oxy)-2,6-difluorophenyl)-1-phenyl-5-(trifluoromethyl)-1H-pyrazole-4-carboxamide |
| Example 9 | 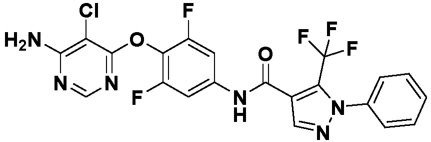 | N-(4-((6-amino-5-chloropyrimidin-4-yl)oxy)-3,5-difluorophenyl)-1-phenyl-5-(trifluoromethyl)-1H-pyrazole-4-carboxamide |
| Example 10 | 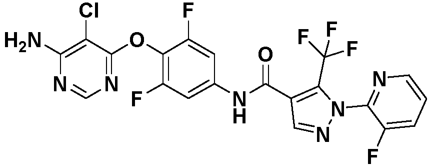 | N-(4-((6-amino-5-chloropyrimidin-4-yl)oxy)-3,5-difluorophenyl)-1-(3-fluoropyridin-2-yl)-5-(trifluoromethyl)-1H-pyrazole-4-carboxamide |
| Example 11 | 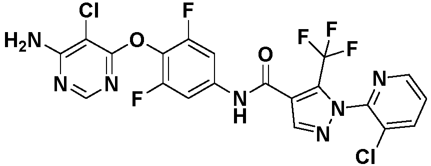 | N-(4-((6-amino-5-chloropyrimidin-4-yl)oxy)-3,5-difluorophenyl)-1-(3-chloropyridin-2-yl)-5-(trifluoromethyl)-1H-pyrazole-4-carboxamide |
| Example 12 | 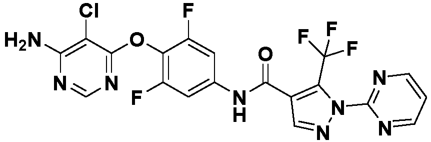 | N-(4-((6-amino-5-chloropyrimidin-4-yl)oxy)-3,5-difluorophenyl)-1-(pyrimidin-2-yl)-5-(trifluoromethyl)-1H-pyrazole-4-carboxamide |
| Example 13 | 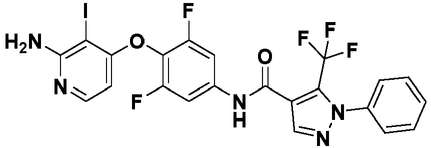 | N-(4-((2-amino-3-iodopyridin-4-yl)oxy)-3,5-difluorophenyl)-1-phenyl-5-(trifluoromethyl)-1H-pyrazole-4-carboxamide |
| Example 14 | 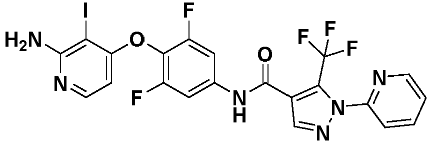 | N-(4-((2-amino-3-iodopyridin-4-yl)oxy)-3,5-difluorophenyl)-1-(pyridin-2-yl)-5-(trifluoromethyl)-1H-pyrazole-4-carboxamide |
| Example 15 | 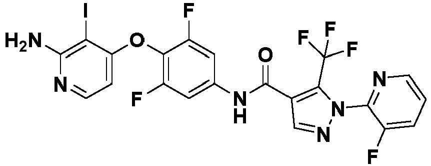 | N-(4-((2-amino-3-iodopyridin-4-yl)oxy)-3,5-difluorophenyl)-1-(3-fluoropyridin-2-yl)-5-(trifluoromethyl)-1H-pyrazole-4-carboxamide |
| Example 16 | 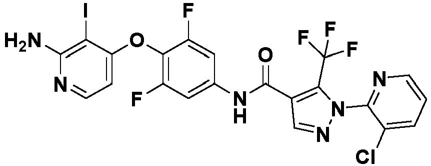 | N-(4-((2-amino-3-iodopyridin-4-yl)oxy)-3,5-difluorophenyl)-1-(3-chloropyridin-2-yl)-5-(trifluoromethyl)-1H-pyrazole-4-carboxamide |
| Example 17 | 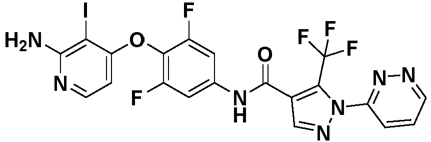 | N-(4-((2-amino-3-iodopyridin-4-yl)oxy)-3,5-difluorophenyl)-1-(pyridazin-3-yl)-5-(trifluoromethyl)-1H-pyrazole-4-carboxamide |
| Example 18 | 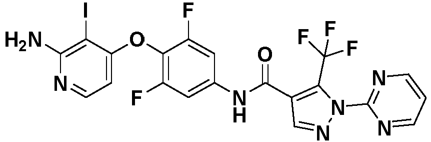 | N-(4-((2-amino-3-iodopyridin-4-yl)oxy)-3,5-difluorophenyl)-1-(pyrimidin-2-yl)-5-(trifluoromethyl)-1H-pyrazole-4-carboxamide |
| Example 19 | 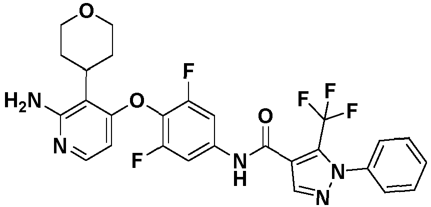 | N-(4-((2-amino-3-(tetrahydro-2H-pyran-4-yl)pyridin-4-yl)oxy)-3,5-difluorophenyl)-1-phenyl-5-(trifluoromethyl)-1H-pyrazole-4-carboxamide; |
| Example 20 | 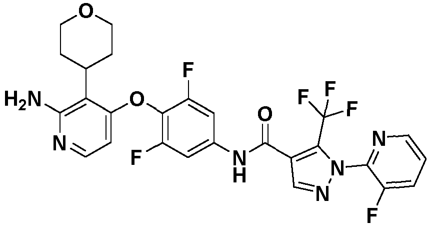 | N-(4-((2-amino-3-(tetrahydro-2H-pyran-4-yl)pyridin-4-yl)oxy)-3,5-difluorophenyl)-1-(3-fluoropyridin-2-yl)-5-(trifluoromethyl)-1H-pyrazole-4-carboxamide |
| Example 21 | 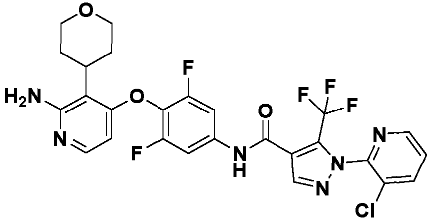 | N-(4-((2-amino-3-(tetrahydro-2H-pyran-4-yl)pyridin-4-yl)oxy)-3,5-difluorophenyl)-1-(3-chloropyridin-2-yl)-5-(trifluoromethyl)-1H-pyrazole-4-carboxamide |
| Example 22 | 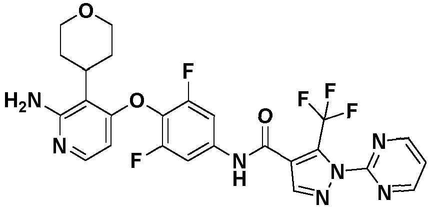 | N-(4-((2-amino-3-(tetrahydro-2H-pyran-4-yl)pyridin-4-yl)oxy)-3,5-difluorophenyl)-1-(pyrimidin-2-yl)-5-(trifluoromethyl)-1H-pyrazole-4-carboxamide |
| Example 23 | 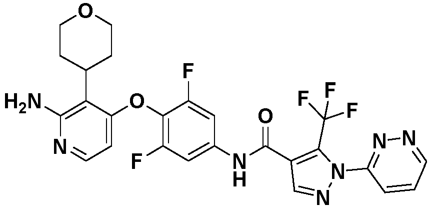 | N-(4-((2-amino-3-(tetrahydro-2H-pyran-4-yl)pyridin-4-yl)oxy)-3,5-difluorophenyl)-1-(pyridazin-3-yl)-5-(trifluoromethyl)-1H-pyrazole-4-carboxamide |
| Example 24 | 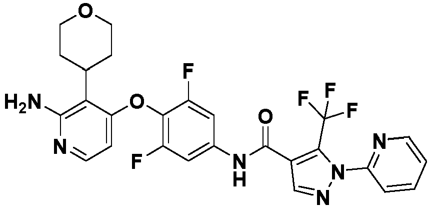 | N-(4-((2-amino-3-(tetrahydro-2H-pyran-4-yl)pyridin-4-yl)oxy)-3,5-difluorophenyl)-1-(pyridin-2-yl)-5-(trifluoromethyl)-1H-pyrazole-4-carboxamide |
| Example 25 | 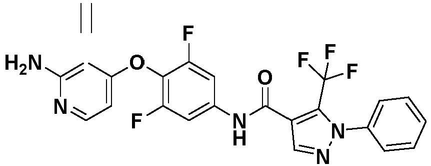 | N-(4-((2-amino-3-ethynylpyridin-4-yl)oxy)-3,5-difluorophenyl)-1-phenyl-5-(trifluoromethyl)-1H-pyrazole-4-carboxamide |
| Example 26 | 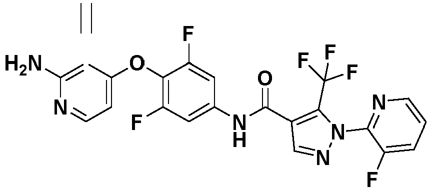 | N-(4-((2-amino-3-ethynylpyridin-4-yl)oxy)-3,5-difluorophenyl)-1-(3-fluoropyridin-2-yl)-5-(trifluoromethyl)-1H-pyrazole-4-carboxamide |
| Example 27 | 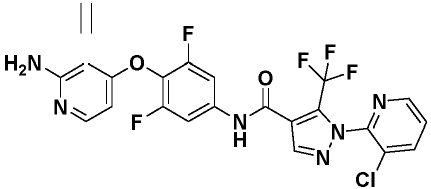 | N-(4-((2-amino-3-ethynylpyridin-4-yl)oxy)-3,5-difluorophenyl)-1-(3-chloropyridin-2-yl)-5-(trifluoromethyl)-1H-pyrazole-4-carboxamide |
| Example 28 | 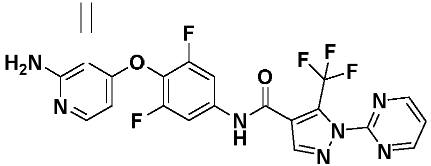 | N-(4-((2-amino-3-ethynylpyridin-4-yl)oxy)-3,5-difluorophenyl)-1-(pyrimidin-2-yl)-5-(trifluoromethyl)-1H-pyrazole-4-carboxamide |
| Example 29 | 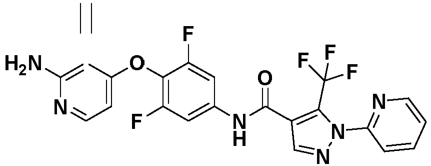 | N-(4-((2-amino-3-ethynylpyridin-4-yl)oxy)-3,5-difluorophenyl)-1-(pyridin-2-yl)-5-(trifluoromethyl)-1H-pyrazole-4-carboxamide |
| Example 30 | 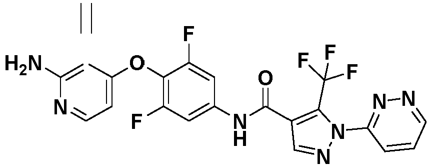 | N-(4-((2-amino-3-ethynylpyridin-4-yl)oxy)-3,5-difluorophenyl)-1-(pyridazin-3-yl)-5-(trifluoromethyl)-1H-pyrazole-4-carboxamide |
| Example 31 | 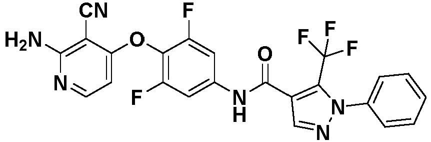 | N-(4-((2-amino-3-cyanopyridin-4-yl)oxy)-3,5-difluorophenyl)-1-phenyl-5-(trifluoromethyl)-1H-pyrazole-4-carboxamide |
| Example 32 | 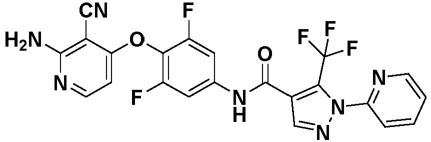 | N-(4-((2-amino-3-cyanopyridin-4-yl)oxy)-3,5-difluorophenyl)-1-(pyridin-2-yl)-5-(trifluoromethyl)-1H-pyrazole-4-carboxamide |
| Example 33 | 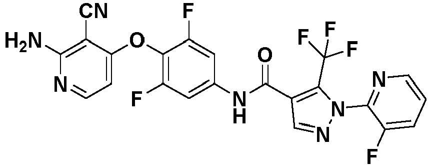 | N-(4-((2-amino-3-cyanopyridin-4-yl)oxy)-3,5-difluorophenyl)-1-(3-fluoropyridin-2-yl)-5-(trifluoromethyl)-1H-pyrazole-4-carboxamide |
| Example 34 | 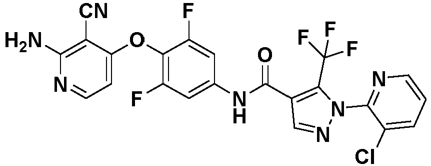 | N-(4-((2-amino-3-cyanopyridin-4-yl)oxy)-3,5-difluorophenyl)-1-(3-chloropyridin-2-yl)-5-(trifluoromethyl)-1H-pyrazole-4-carboxamide |
| Example 35 | 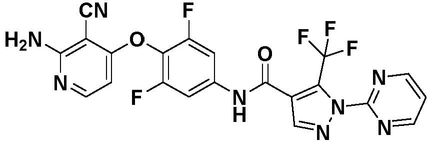 | N-(4-((2-amino-3-cyanopyridin-4-yl)oxy)-3,5-difluorophenyl)-1-(pyrimidin-2-yl)-5-(trifluoromethyl)-1H-pyrazole-4-carboxamide |
| Example 36 | 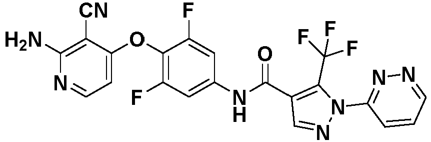 | N-(4-((2-amino-3-cyanopyridin-4-yl)oxy)-3,5-difluorophenyl)-1-(pyridazin-3-yl)-5-(trifluoromethyl)-1H-pyrazole-4-carboxamide |
| Example 37 | 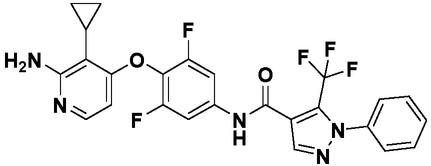 | N-(4-((2-amino-3-cyclopropylpyridin-4-yl)oxy)-3,5-difluorophenyl)-1-phenyl-5-(trifluoromethyl)-1H-pyrazole-4-carboxamide |
| Example 38 | 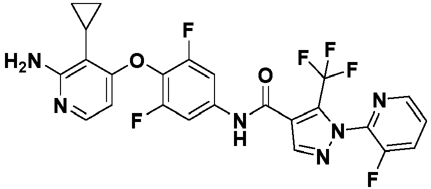 | N-(4-((2-amino-3-cyclopropylpyridin-4-yl)oxy)-3,5-difluorophenyl)-1-(3-fluoropyridin-2-yl)-5-(trifluoromethyl)-1H-pyrazole-4-carboxamide |
| Example 39 | 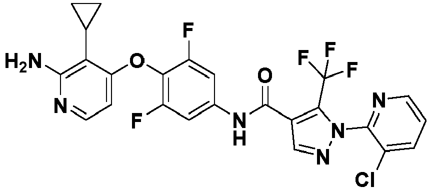 | N-(4-((2-amino-3-cyclopropylpyridin-4-yl)oxy)-3,5-difluorophenyl)-1-(3-chloropyridin-2-yl)-5-(trifluoromethyl)-1H-pyrazole-4-carboxamide |
| Example 40 | 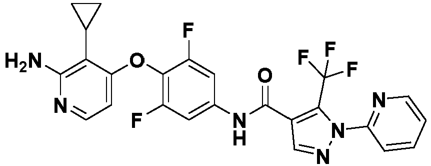 | N-(4-((2-amino-3-cyclopropylpyridin-4-yl)oxy)-3,5-difluorophenyl)-1-(pyridin-2-yl)-5-(trifluoromethyl)-1H-pyrazole-4-carboxamide |
| Example 41 | 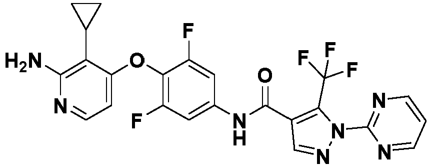 | N-(4-((2-amino-3-cyclopropylpyridin-4-yl)oxy)-3,5-difluorophenyl)-1-(pyrimidin-2-yl)-5-(trifluoromethyl)-1H-pyrazole-4-carboxamide |
| Example 42 | 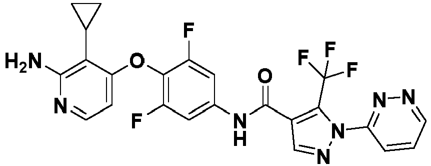 | N-(4-((2-amino-3-cyclopropylpyridin-4-yl)oxy)-3,5-difluorophenyl)-1-(pyridazin-3-yl)-5-(trifluoromethyl)-1H-pyrazole-4-carboxamide |
| Example 43 | 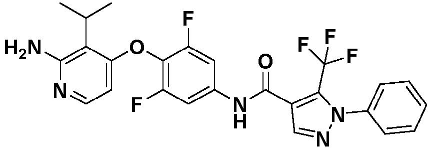 | N-(4-((2-amino-3-isopropylpyridin-4-yl)oxy)-3,5-difluorophenyl)-1-phenyl-5-(trifluoromethyl)-1H-pyrazole-4-carboxamide |
| Example 44 | 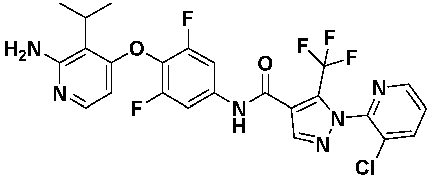 | N-(4-((2-amino-3-isopropylpyridin-4-yl)oxy)-3,5-difluorophenyl)-1-(3-chloropyridin-2-yl)-5-(trifluoromethyl)-1H-pyrazole-4-carboxamide |
| Example 45 | 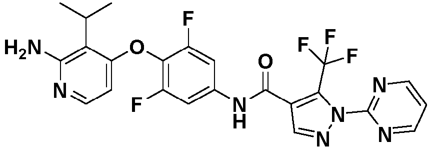 | N-(4-((2-amino-3-isopropylpyridin-4-yl)oxy)-3,5-difluorophenyl)-1-(pyrimidin-2-yl)-5-(trifluoromethyl)-1H-pyrazole-4-carboxamide |
| Example 46 | 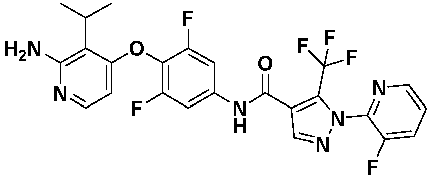 | N-(4-((2-amino-3-isopropylpyridin-4-yl)oxy)-3,5-difluorophenyl)-1-(3-fluoropyridin-2-yl)-5-(trifluoromethyl)-1H-pyrazole-4-carboxamide |
| Example 47 | 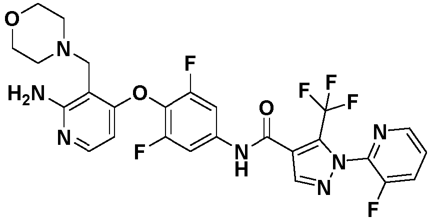 | N-(4-((2-amino-3-(morpholinomethyl)pyridin-4-yl)oxy)-3,5-difluorophenyl)-1-(3-fluoropyridine-2-yl)-5-(trifluoromethyl)-1H-pyrazole-4-carboxamide |
| Example 48 | 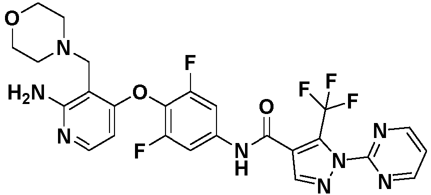 | N-(4-((2-amino-3-(morpholinomethyl)pyridin-4-yl)oxy)-3,5-difluorophenyl)-1-(pyrimidin-2-yl)-5-(trifluoromethyl)-1H-pyrazole-4-carboxamide |
| Example 49 | 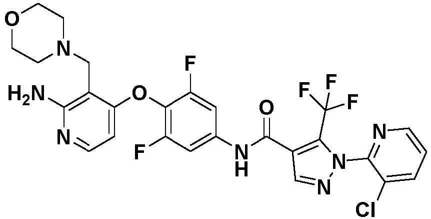 | N-(4-((2-amino-3-(morpholinomethyl)pyridin-4-yl)oxy)-3,5-difluorophenyl)-1-(3-chloropyridin-2-yl)-5-(trifluoromethyl)-1H-pyrazole-4-carboxamide |
| Example 50 | 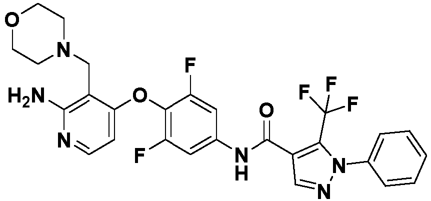 | N-(4-((2-amino-3-(morpholinomethyl)pyridin-4-yl)oxy)-3,5-difluorophenyl)-1-phenyl-5-(trifluoromethyl)-1H-pyrazole-4-carboxamide |
| Example 51 | 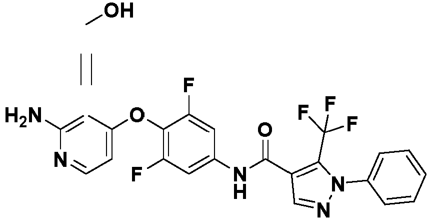 | N-(4-((2-amino-3-(3-hydroxyprop-1-yn-1-yl)pyridin-4-yl)oxy)-3,5-difluorophenyl)-1-phenyl-5-(trifluoromethyl)-1H-pyrazole-4-carboxamide |
| Example 52 | 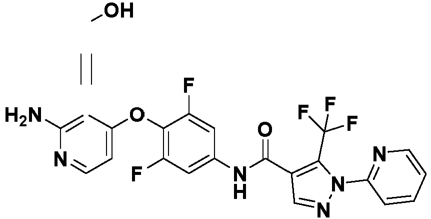 | N-(4-((2-amino-3-(3-hydroxyprop-1-yn-1-yl)pyridin-4-yl)oxy)-3,5-difluorophenyl)-1-(pyridin-2-yl)-5-(trifluoromethyl)-1H-pyrazole-4-carboxamide |
| Example 53 | 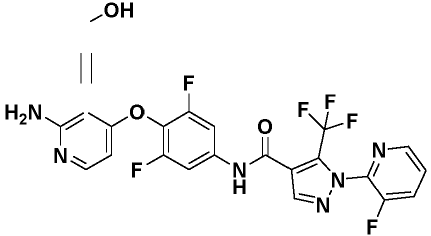 | N-(4-((2-amino-3-(3-hydroxyprop-1-yn-1-yl)pyridin-4-yl)oxy)-3,5-difluorophenyl)-1-(3-fluoropyridin-2-yl)-5-(trifluoromethyl)-1H-pyrazole-4-carboxamide |
| Example 54 | 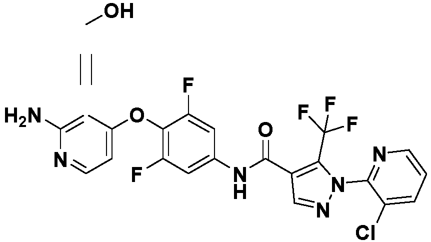 | N-(4-((2-amino-3-(3-hydroxyprop-1-yn-1-yl)pyridin-4-yl)oxy)-3,5-difluorophenyl)-1-(3-chloropyridin-2-yl)-5-(trifluoromethyl)-1H-pyrazole-4-carboxamide |
| Example 55 | 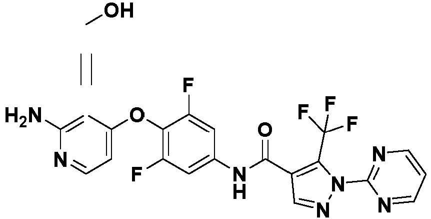 | N-(4-((2-amino-3-(3-hydroxyprop-1-yn-1-yl)pyridin-4-yl)oxy)-3,5-difluorophenyl)-1-(pyrimidin-2-yl)-5-(trifluoromethyl)-1H-pyrazole-4-carboxamide |
| Example 56 | 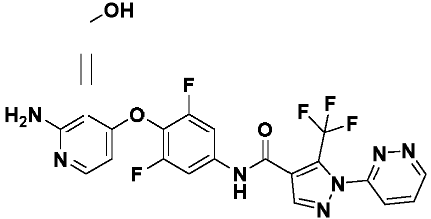 | N-(4-((2-amino-3-(3-hydroxyprop-1-yn-1-yl)pyridin-4-yl)oxy)-3,5-difluorophenyl)-1-(pyridazin-3-yl)-5-(trifluoromethyl)-1H-pyrazole-4-carboxamide |
| Example 57 | 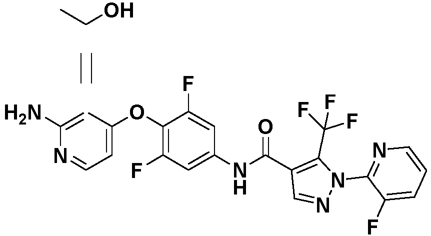 | N-(4-((2-amino-3-(3-hydroxybut-1-yn-1-yl)pyridin-4-yl)oxy)-3,5-difluorophenyl)-1-(3-fluoropyridin-2-yl)-5-(trifluoromethyl)-1H-pyrazole-4-carboxamide |
| Example 58 | 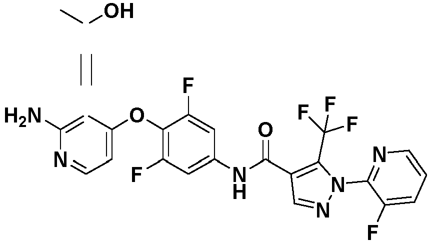 | N-(4-((2-amino-3-(3-hydroxy-3-methylbut-1-yn-1-yl)pyridin-4-yl)oxy)-3,5-difluorophenyl)-1-(3-fluoropyridin-2-yl)-5-(trifluoromethyl)-1H-pyrazole-4-carboxamide |
| Example 59 | 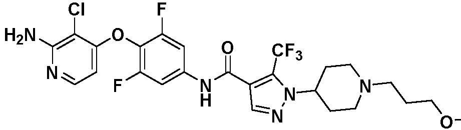 | N-(4-((2-amino-3-chloropyridin-4-yl)oxy)-3,5-difluorophenyl)-1-(1-(3-methoxypropyl)piperi din-4-yl)-5-(trifluoromethyl)-1H-pyrazole-4-carboxamide |
| Example 60 | 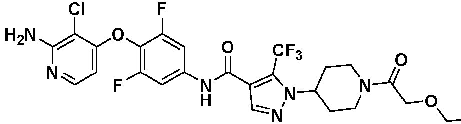 | N-(4-((2-amino-3-chloropyridin-4-yl)oxy)-3,5-difluorophenyl)-1-(1-(2-ethoxyacetyl)piperi din-4-yl)-5-(trifluoromethyl)-1H-pyrazole-4-carboxamide |
| Example 61 | 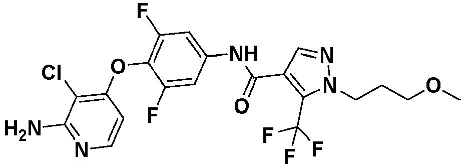 | N-[4-[(2-amino-3-chloro-4-pyridyl)oxy]-3,5-difluorophenyl]-1-(3-methoxypropyl)-5-(trifluoromethyl)pyrazole-4-carboxamide |

### 2. Use of novel compound

The novel compound of the present invention has the activity of inhibiting tyrosine protein kinases, such as RON, which increase the proliferation, invasion and metastasis of tumor cells, and enhance the resistance of tumor cells against cell apoptosis and cytotoxic therapy.

Therefore, the novel compound of the present invention can be used as an active ingredient of a pharmaceutical composition. Accordingly, another aspect of the present invention provides a pharmaceutical composition including the above compound as an active ingredient.

The pharmaceutical composition may further include a pharmaceutically acceptable carrier.

The pharmaceutical composition may be useful for preventing or treating protein kinase-mediated diseases, but the use of the present invention is not limited to these diseases.

The pharmaceutical composition may be useful for preventing or treating RON-mediated diseases, but the use of the present invention is not limited to these diseases.

The pharmaceutical composition according to the present invention includes a therapeutically effective amount of the compound of Chemical Formula 1 or a pharmaceutically acceptable salt thereof.

For the treatment of the above diseases, the pharmaceutical composition may be administered to a subject in need of prevention or treatment of the diseases.

The pharmaceutical composition according to the present invention may be administered orally or parenterally during clinical administration and may be used in the form of a general pharmaceutical preparation. In other words, the pharmaceutical composition of the present invention may be administered in various oral and parenteral dosage forms during actual clinical administration. When it is formulated, diluents or excipients, such as commonly used fillers, extenders, binders, wetting agents, disintegrants, and surfactants, are used for formulation. Solid preparations for oral administration include tablets, pills, powder, granules, and capsules, and these solid preparations are formulated by mixing at least one excipient, for example, starch, calcium carbonate, sucrose or lactose and gelatin with a herbal extract or herbal fermentation product. Furthermore, in addition to simple excipients, lubricants such as magnesium stearate, talc are also used. Liquid preparations for oral administration include suspensions, oral solutions, emulsions, and syrups. In addition to commonly used simple diluents such as water and liquid paraffin, various excipients such as wetting agents, sweeteners, fragrances, and preservatives may be included. Preparations for parenteral administration include sterilized aqueous solutions, non-aqueous solutions, suspensions, emulsions, freeze-dried preparations, and suppositories. As non-aqueous solvents and suspensions, propylene glycol, polyethylene glycol, vegetable oils such as olive oil, and an injectable ester such as ethyl oleate may be used. As a base for suppositories, Witepsol, Macrogol, Tween 61, cacao oil, laurel oil, glycerol, gelatin or the like may be used.

When the pharmaceutical composition is administered for clinical purposes, the effective dosage of the compound of Chemical Formula 1 or a pharmaceutically acceptable salt thereof may vary depending on factors such as the formulation method, administration method, a patient's age, weight, sex, pathological conditions, diet, administration time, route of administration, excretion rate, drug mixing, and response sensitivity, but it is generally 0.01 to 20 mg/day per kg of body weight in an adult patient, preferably 1 to 10 mg/day, and the composition may be administered in divided doses several times a day, preferably 2 to 3 times a day, at regular intervals according to the judgment of a doctor or pharmacist.

In still another aspect, the present invention provides a method of treating protein kinase-mediated diseases, such as RON-mediated diseases, including administering a therapeutically effective amount of the compound of Chemical Formula 1 or a pharmaceutically acceptable salt thereof to a subject.

In yet another aspect, the present invention provides a method of inhibiting RON receptor activity, including administering a therapeutically effective amount of the compound of Chemical Formula 1 or a pharmaceutically acceptable salt thereof to a subject.

Hereinafter, the example compounds of the present invention may be prepared according to the method described below, but the compounds of the present invention are not limited by the production method.

### Preparation Example 1. 4-(4-Amino-2,6-difluorophenoxy)-3-chloropyridin-2-amine

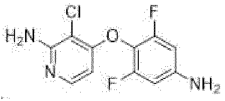

The compound of Preparation Example 1 was prepared according to Scheme 1 below.

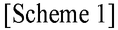

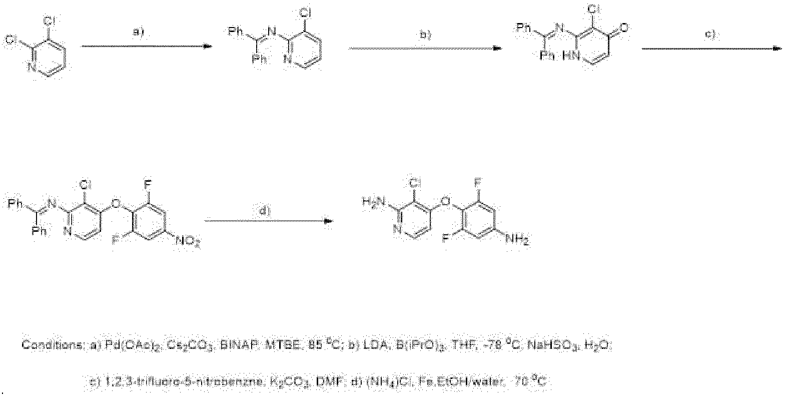

### Step 1) N-(3-chloropyridin-2-yl)-1,1-diphenylmethanimine

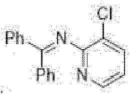

2,3-dichloropyridine (21 g, 142 mmol) was dissolved in methyl-tert-butyl ester (MTBE), and then Pd(OAc)₂ (318 mg, 1.42 mmol), rac-BINAP (1.4 g, 2.13 mmol), Cs₂CO₃ (0.9 g, 2.84 mmol), and benzophenone imine (26 g, 142 mmol) were added. The reaction mixture was stirred under reflux at 70 °C for 12 h. The reaction mixture was cooled to room temperature, the resulting solid was filtered through a Celite filter, and the solvent was concentrated. The residue was purified by column chromatography to obtain the title compound (20 g, yield: 48%).
MS m/z : 293[M+H]

### Step 2) 3-chloro-2-((diphenylmethylene)amino)pyridin-4(1H)-one

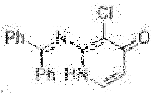

The compound N-(3-chloropyridin-2-yl)-1,1-diphenylmethanimine (20 g, 68.3 mmol) obtained in Step 1 was dissolved in 100 ml of THF, and then tri-isopropylborate (19.3 g, 102 mmol) was added, and the resulting mixture was cooled to 0 °C. Lithium diisopropylamide (11 mL, 88.8 mmol) was slowly added to the reaction mixture at 0 °C. The reaction mixture was stirred at 0 °C for two hours, 100 ml of water was added, sodium percarbonate (16 g, 102 mmol) was added, and then the resulting mixture was further stirred at room temperature for three hours. 50 ml of saturated NaHSO₃ was slowly added to the reaction mixture, and the resulting mixture was extracted three times with EA. The organic layer was concentrated and used in Step 3 below without purification.
MS m/z : 309[M+H]

### Step 3) N-(3-chloro-4-(2,6-difluoro-4-nitrophenoxy)pyridin-2-yl)-1,1-diphenylmethanimine

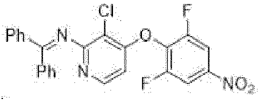

The compound 3-chloro-2-((diphenylmethylene)amino)pyridin-4(1H)-one (60 g, 194 mmol) obtained in Step 2 was dissolved in a DMF solution, 1,2,3-trifluoro-5-nitrobenzene (24.9 ml, 214 mmol), and Cs₂CO₃ (31.7 g, 97 mmol) were added, and the resulting mixture was stirred at room temperature for 12 h. Ice was added to the reaction mixture, and the resulting solid was filtered. The solid was washed with methanol and dried to obtain the title compound (53 g, yield: 58%).
MS m/z : 466[M+H]

### Step 4) 4-(4-amino-2,6-difluorophenoxy)-3-chloropyridin-2-amine

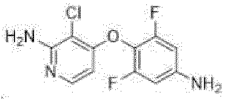

The compound N-(3-chloro-4-(2,6-difluoro-4-nitrophenoxy)pyridin-2-yl)-1,1-diphenylmethanimine (14 g, 29.6 mmol) obtained in Step 3 was dissolved in ethanol/water (4:1), iron powder (5.8 g, 104 mmol) and NH₄Cl (5.6 g, 104 mmol) were added, and the resulting mixture was stirred at 80 °C for six hours. After cooling to room temperature, the solid was filtered, and the filtrate was washed with water. After concentrating the organic layer, DCM was added, and the resulting solid was filtered and dried to obtain the title compound (5.6 g, 70%).
MS m/z : 272[M+H]

### Preparation Example 2. 4-(4-amino-2,5-difluorophenoxy)-3-chloropyridin-2-amine

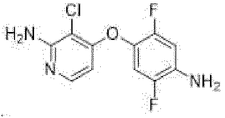

According to Preparation Example 1, 1,3,5-trifluoro-2-nitrobenzene was used instead of 1,2,3-trifluoro-5-nitrobenzene to obtain the title compound (220 mg, yield: 45%) in a manner similar to Preparation Example 1.
MS m/z : 272[M+H]

### Preparation Example 3. 4-(4-amino-3,5-difluorophenoxy)-3-chloropyridin-2-amine

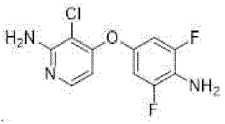

According to Preparation Example 1, 1,3,5-trifluoro-2-nitrobenzene was used instead of 1,2,3-trifluoro-5-nitrobenzene to obtain the title compound (190 mg, yield: 55%) in a manner similar to Preparation Example 1.
MS m/z : 272[M+H]

### Preparation Example 4. 6-(4-amino-2,6-difluorophenoxy)-5-chloropyrimidin-4-amine

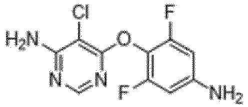

Hereinafter, a compound of Preparation Example 4 was prepared according to Scheme 2 below.

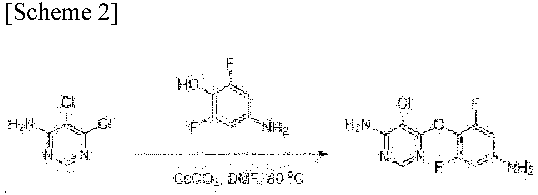

5,6-Dichloropyrimidin-4-amine (300 mg, 1.83 mmol) was dissolved in 5 ml of DMF, 4-amino-2,6-difluorophenol (265 mg, 1.83 mmol) and Cs₂CO₃ (894 mg, 2.74 mmol) were added. The reaction mixture was stirred at 80 °C for one hour. After cooling to room temperature, the reaction mixture was filtered through a Celite filter, and the filtrate was concentrated. The residue was purified by column chromatography to obtain the title compound (154 mg, 31%).
¹H NMR (500 MHz, DMSO-d6) δ 7.97 (d, 1H), 7.40 (brs, 2H), 6.31 (m, 2H), 5.68 (brs, 2H); MS m/z : 273[M+H]

### Preparation Example 5. 4-(4-amino-2,6-difluorophenoxy)-3-iodopyridin-2-amine

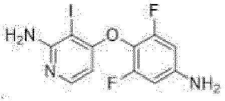

Hereinafter, a compound of Preparation Example 5 was prepared according to Scheme 3 below.

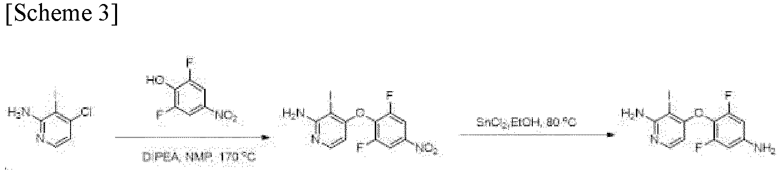

### Step 1) 4-(2,6-difluoro-4-nitrophenoxy)-3-iodopyridin-2-amine

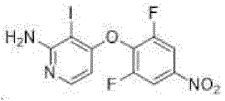

4-Chloro-3-iodopyridin-2-amine (13 g, 51.1 mmol) was dissolved in 500 ml of N-methyl-2-pyrrolidone (NMP), and then 2,6-difluoro-4-nitrophenol (13.5 g, 76.6 mmol) and DIPEA (17.8 ml, 102 mmol) were added. The reaction mixture was stirred under reflux at 170 °C for 12 h. The reaction mixture was cooled to room temperature, diluted with water and extracted three times with EA. The organic solution was washed with brine and concentrated. The residue was purified by column chromatography to obtain the title compound (6.4 g, yield: 32%).
MS m/z : 394[M+H]

### Step 2) 4-(4-amino-2,6-difluorophenoxy)-3-iodopyridin-2-amine

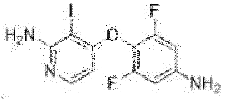

The compound 4-(2,6-difluoro-4-nitrophenoxy)-3-iodopyridin-2-amine (6 g, 15.3 mmol) obtained in Step 1 was dissolved in 300 ml of ethanol, and then SnCl₂ (11.6 g, 61.2 mmol) was added. The reaction mixture was heated to 80 °C and stirred for four hours. After cooling to room temperature, ethanol was concentrated and diluted with EA. A saturated aqueous NaHCO₃ solution was added, the resulting mixture was extracted three times with EA, the organic layer was concentrated, and then the residue was purified by column chromatography to obtain the title compound (4.2 g, yield: 76%).
¹H NMR (500 MHz, DMSO-d6) δ 7.74 (d, 1H), 6.37 (d, 2H), 6.17 (brs, 2H), 5.79 (m, 3H); MS m/z : 364[M+H]

### Preparation Example 6. 1-(3-fluoropyridin-2-yl)-5-(trifluoromethyl)-1H-pyrazole-4-carboxylic acid

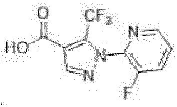

Hereinafter, a compound of Preparation Example 6 was prepared according to Scheme 4 below.

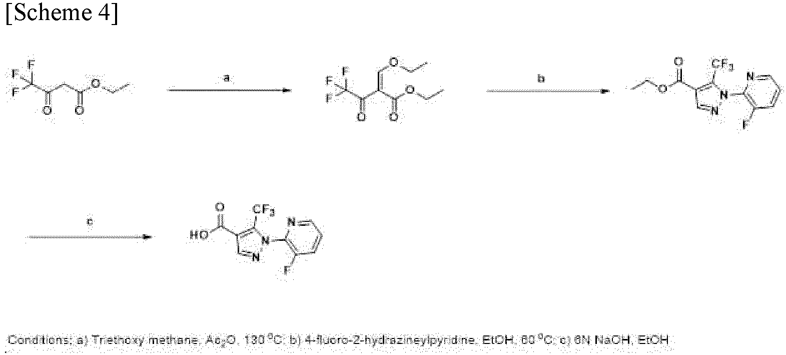

### Step 1) Ethyl 2-(ethoxymethylene)-4,4,4-trifluoro-3-oxobutanoate

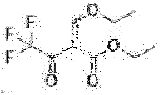

Ethyl 4,4,4-trifluoro-3-oxobutanoate (2.0 g, 10.9 mmol) and triethoxy methane (2.0 g, 14.1 mmol) were dissolved in acetic anhydride (3.3 g, 32.6 mmol), and the resulting mixture was stirred at 130 °C for four hours. The reaction mixture was concentrated to obtain the title compound (2.0 g, yield: 77%).
MS m/z : 241[M+H].

### Step 2) Ethyl 1-(3-fluoropyridin-2-yl)-5-(trifluoromethyl)-1H-pyrazole-4-carboxylate

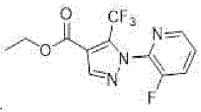

The compound ethyl 2-(ethoxymethylene)-4,4,4-trifluoro-3-oxobutanoate (0.5 g, 2.08 mmol) obtained in Step 1 and 4-fluoro-2-hydrazineylpyridine (0.24 g, 1.87 mmol) were dissolved in 6 ml of ethanol, the resulting mixture was stirred at 60 °C for 12 h, and then the reaction mixture was extracted using EA and water. The residue was purified by column chromatography to obtain the title compound (0.35 g, yield: 55%).
MS m/z : 304[M+H].

### Step 3) 1-(3-fluoropyridin-2-yl)-5-(trifluoromethyl)-1H-pyrazole-4-carboxylic acid

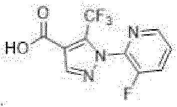

The compound ethyl 1-(3-fluoropyridin-2-yl)-5-(trifluoromethyl)-1H-pyrazole-4-carboxylate (0.35 g, 2.01 mmol) obtained in Step 2 was dissolved in 4 ml of ethanol, 2 ml of 6 N sodium hydroxide solution was added at room temperature, and the resulting mixture was stirred for one hour. Ice water was added to the reaction product, and the resulting solid was filtered to obtain the title compound (0.26 g, yield: 82%).

MS m/z : 276[M+H].

### Example 1. N-(4-((2-amino-3-chloropyridin-4-yl)oxy)-3,5-difluorophenyl)-1-phenyl-5-(trifluoromethyl)-1H-pyrazole-4-carboxamide

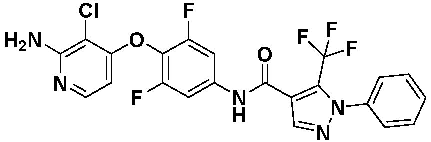

Hereinafter, a compound of Example 1 was prepared according to Scheme 5 below.

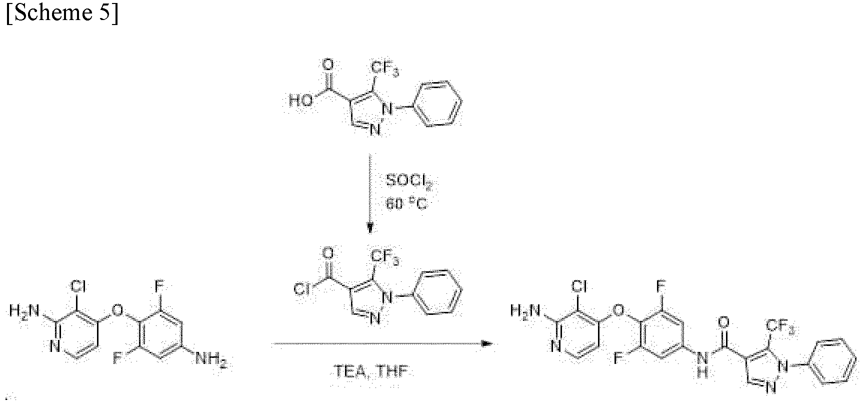

### Step 1) 1-phenyl-5-(trifluoromethyl)-1H-pyrazole-4-carbonyl chloride

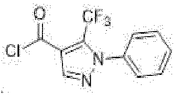

2 ml of SOCl₂ was added to 1-phenyl-5-(trifluoromethyl)-1H-pyrazole-4-carboxylic acid (46 mg, 0.18 mmol), and the resulting mixture was stirred at 60 °C for one hour. The reaction mixture was cooled to room temperature and concentrated to obtain 1-phenyl-5-(trifluoromethyl)-1H-pyrazole-4-carbonyl chloride.

### Step 2) N-(4-((2-amino-3-chloropyridin-4-yl)oxy)-3,5-difluorophenyl)-1-phenyl-5-(trifluoromethyl)-1H-pyrazole -4-carboxamide

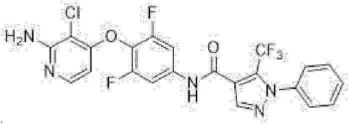

The compound 4-(4-amino-2,6-difluorophenoxy)-3-chloropyridin-2-amine (40 mg, 0.15 mmol) obtained in Preparation Example 1 was dissolved in 3 ml of THF, 1-phenyl-5-(trifluoromethyl)-1H-pyrazole-4-carbonyl chloride (49 mg, 0.18 mmol) obtained in Step 1 and TEA (31 µl, 0.22 mmol) were added, and the resulting mixture was stirred at room temperature for 12 h. The reaction solution was diluted with water, extracted three times with EA, and concentrated. The residue was purified by prep-HPLC (0.1% formic acid in water/acetonitrile) to obtain the title compound (53 mg, yield: 71%).
¹H NMR (500 MHz, DMSO-d₆) δ 11.01 (s, 1H), 8.35 (s, 1H), 7.82 (d, 1H), 7.70 (m, 6H), 6.84 (brs, 2H), 6.15 (d, 1H); MS m/z : 511[M+H]

### Example 2. N-(4-((2-amino-3-chloropyridin-4-yl)oxy)-3,5-difluorophenyl)-1-(pyrimidin-2-yl)-5-(trifluoromethyl)-1H-pyrazole-4-carboxamide

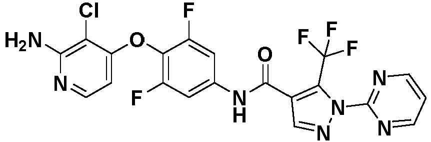

According to Example 1, 1-(pyrimidin-2-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (14 mg, yield: 28%) in a manner similar to Example 1.
¹H NMR (500 MHz, DMSO-d₆) δ 11.11 (s, 1H), 9.47 (d, 1H), 8.52 (s, 1H), 8.26 (d, 1H), 8.12 (m, 1H), 7.78 (d, 1H), 7.67 (m, 2H), 6.50 (s, 2H), 6.06 (d, 1H); MS m/z : 513[M+H]

### Example 3. N-(4-((2-amino-3-chloropyridin-4-yl)oxy)-3,5-difluorophenyl)-1-(pyridazin-3-yl)-5-(trifluoro Methyl)-1H-pyrazole-4-carboxamide

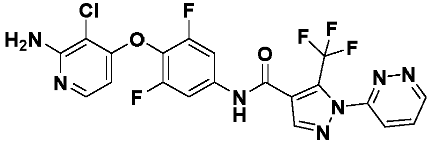

According to Example 1, 1-(pyridazin-3-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (11 mg, yield: 20%) in a manner similar to Example 1.
¹H NMR (500 MHz, DMSO-d₆) δ 11.09 (s, 1H), 9.10 (d, 1H), 8.43 (s, 1H), 7.82 (d, 2H), 7.67 (m, 2H), 6.52 (s, 2H), 6.06 (d, 1H); MS m/z : 513[M+H]

### Example 4. N-(4-((2-amino-3-chloropyridin-4-yl)oxy)-3,5-difluorophenyl)-5-ethyl-1-(3-fluoropyridin-2-yl)-1H-pyrazole-4-carboxamide

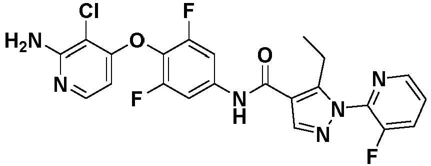

According to Example 1, 5-ethyl-1-(3-fluoropyridin-2-yl)-1H-pyrazole-4-carbonyl chloride was used instead of 1-phenyl-5-(trifluoromethyl)-1H-pyrazole-4-carbonyl chloride to obtain the title compound (30 mg, yield: 40%) in a manner similar to Example 1.
¹H NMR (500 MHz, DMSO-d₆) δ 10.36 (s, 1H), 8.54 (d, 1H), 8.37 (s, 1H), 8.16 (t, 1H), 7.78 (m, 4H), 6.49 (s, 2H), 6.04 (d, 1H), 2.34 (q, 2H), 1.08 (t, 3H); MS m/z : 489[M+H]

### Example 5. N-(4-((2-amino-3-chloropyridin-4-yl)oxy)-2,5-difluorophenyl)-1-(3-fluoropyridin-2-yl)-5-(trifluoromethyl)-1H-pyrazole-4-carboxamide

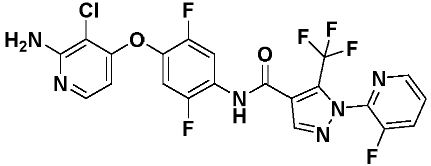

According to Example 1, 4-(4-amino-2,5-difluorophenoxy)-3-chloropyridin-2-amine obtained in Preparation Example 2 was used instead of 4-(4-amino-2,6-difluorophenoxy)-3-chloropyridin-2-amine to obtain the title compound (28 mg, yield: 61%) in a manner similar to Example 1.
¹H NMR (500 MHz, DMSO-d₆) δ 10.72 (s, 1H), 8.56 (d, 1H), 8.47 (d, 1H), 8.21 (t, 1H), 7.98 (m, 1H), 7.87 (m, 1H), 7.78 (m, 1H), 7.55 (t, 1H), 6.48 (brs, 2H), 6.08 (brs, 1H); MS m/z : 529[M+H]

### Example 6. N-(4-((2-amino-3-chloropyridin-4-yl)oxy)-2,5-difluorophenyl)-1-phenyl-5-(trifluoromethyl)-1H-pyrazole-4-carboxamide

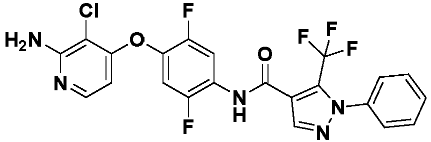

According to Example 5, 1-phenyl-5-(trifluoromethyl)-1H-pyrazole-4-carbonyl chloride was used instead of 1-(3-fluoropyridin-2-yl)-5-(trifluoromethyl)-1H-pyrazole-4-carbonyl chloride to obtain the title compound (22 mg, yield: 57%) in a manner similar to Example 5.
¹H NMR (500 MHz, DMSO-d6) δ 10.62 (s, 1H), 8.32 (s, 1H), 7.97 (m,1H), 7.80 (d, 1H), 7.63 (m, 3H), 7.55 (m, 3H), 6.48 (brs, 2H), 6.07 (brs, 1H); MS m/z : 510[M+H]

### Example 7. N-(4-((2-amino-3-chloropyridin-4-yl)oxy)-2,6-difluorophenyl)-1-(3-fluoropyridin-2-yl)-5-(trifluoromethyl)-1H-pyrazole-4-carboxamide

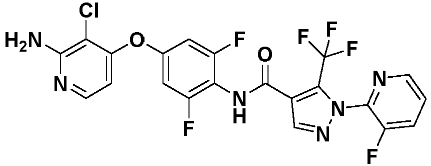

According to Example 1, 4-(4-amino-3,5-difluorophenoxy)-3-chloropyridin-2-amine obtained in Preparation Example 3 was used instead of 4-(4-amino-2,6-difluorophenoxy)-3-chloropyridin-2-amine to obtain the title compound (14 mg, yield: 42%) in a manner similar to Example 1.
¹H NMR (500 MHz, DMSO-d₆) δ 10.48 (s, 1H), 8.56 (d, 1H), 8.49 (d, 1H), 8.23 (t, 1H), 7.88 (m, 2H), 7.14 (m, 2H), 6.52 (brs, 2H), 6.26 (d, 1H); MS m/z : 529[M+H]

### Example 8. N-(4-((6-amino-5-chloropyridin-4-yl)oxy)-3,5-difluorophenyl)-1-phenyl-5-(trifluoromethyl)-1H-pyrazole-4-carboxamide

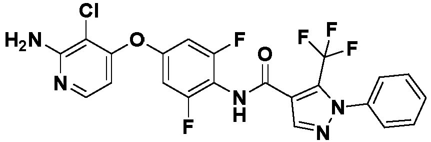

According to Example 7, 1-phenyl-5-(trifluoromethyl)-1H-pyrazole-4-carbonyl chloride was used instead of 1-(3-fluoropyridin-2-yl)-5-(trifluoromethyl)-1H-pyrazole-4-carbonyl chloride to obtain the title compound (16 mg, yield: 46%) in a manner similar to Example 7.
¹H NMR (500 MHz, DMSO-d₆) δ 10.36 (s, 1H), 8.34 (s, 1H), 7.88 (m, 1H), 7.62 (m, 3H), 7.56 (m, 2H), 7.13 (m, 2H), 6.52 (brs, 2H), 6.26 (brs, 1H); MS m/z : 510[M+H]

### Example 9. N-(4-((6-amino-5-chloropyrimidin-4-yl)oxy)-3,5-difluorophenyl)-1-phenyl-5-(trifluoromethyl)-1H-pyrazole-4-carboxamide

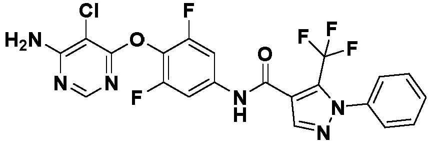

According to Example 1, the compound 6-(4-amino-2,6-difluorophenoxy)-5-chloropyrimidin-4-amine obtained in Preparation Example 4 was used instead of 4-(4-amino-2,6-difluorophenoxy)-3-chloropyridin-2-amine to obtain the title compound (35 mg, yield: 62%) in a manner similar to Example 1.
¹H NMR (500 MHz, DMSO-d6) δ 810.96 (s, 1H), 8.35 (s, 1H), 7.99 (s, 1H), 7.63 - 7.50 (m, 9H); MS m/z : 511[M+H]

### Example 10. N-(4-((6-amino-5-chloropyrimidin-4-yl)oxy)-3,5-difluorophenyl)-1-(3-fluoropyridin-2-yl)-5-(trifluoromethyl)-1H-pyrazole-4-carboxamide

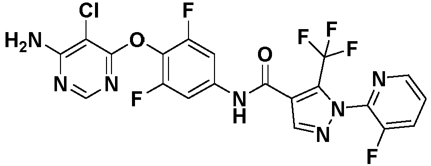

According to Example 9, 1-(3-fluoropyridin-2-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (45 mg, yield: 77%) in a manner similar to Example 9.
¹H NMR (500 MHz, DMSO-d₆) δ 11.00 (s, 1H), 8.56 (d, 1H), 8.49 (m, 1H), 8.23 (t, 1H), 7.99 (d, 1H), 7.88 (m, 1H), 7.62(m, 4H); MS m/z : 530[M+H]

### Example 11. N-(4-((6-amino-5-chloropyrimidin-4-yl)oxy)-3,5-difluorophenyl)-1-(3-chloropyridin-2-yl)-5-(trifluoromethyl)-1H-pyrazole-4-carboxamide

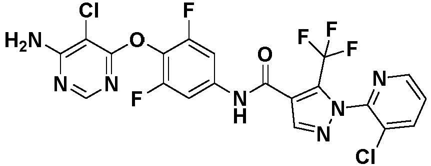

According to Example 9, 1-(3-chloropyridin-2-yl)-5-(trifluoromethyl)-1H-pyrazole-4-carbonyl chloride was used instead of 1-(3-fluoropyridin-2-yl)-5-(trifluoromethyl)-1H-pyrazole-4-carbonyl chloride to obtain the title compound (45 mg, yield: 76%) in a manner similar to Example 9.
¹H NMR (500 MHz, DMSO-d₆) δ 10.97 (s, 1H), 8.68 (brs, 1H), 8.50 (d, 1H), 8.40 (d, 1H), 7.99 (s, 1H), 7.82 (t, 1H), 7.61(m, 4H); MS m/z : 547[M+H]

### Example 12. N-(4-((6-amino-5-chloropyrimidin-4-yl)oxy)-3,5-difluorophenyl)-1-(pyrimidin-2-yl)-5-(trifluoromethyl)-1H-pyrazole-4-carboxamide

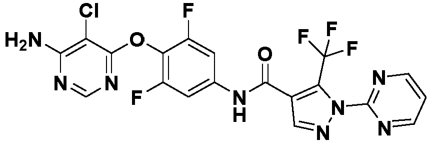

According to Example 9, 1-(pyrimidin-2-yl)-5-(trifluoromethyl)-1H-pyrazole-4-carbonyl chloride was used instead of 1-(3-fluoropyridin-2-yl)-5-(trifluoromethyl)-1H-pyrazole-4-carbonyl chloride to obtain the title compound (40 mg, yield: 71%) in a manner similar to Example 9.
¹H NMR (500 MHz, DMSO-d₆) δ 11.04 (s, 1H), 9.10 (brs, 2H), 8.43 (s, 1H), 7.99 (d, 1H), 7.82 (m, 1H), 7.62(m, 4H); MS m/z : 513[M+H]

### Example 13. N-(4-((2-amino-3-iodopyridin-4-yl)oxy)-3,5-difluorophenyl)-1-phenyl-5-(trifluoromethyl)-1H-pyrazole-4-carboxamide

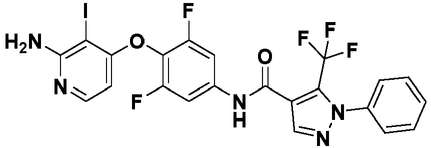

According to Example 1, the compound 4-(4-amino-2,6-difluorophenoxy)-3-iodopyridin-2-amine obtained in Preparation Example 5 was used instead of 4-(4-amino-2,6-difluorophenoxy)-3-chloropyridin-2-amine to obtain the title compound (720 mg, yield: 63%) in a manner similar to Example 1.
¹H NMR (500 MHz, DMSO-d₆) δ 11.00 (s, 1H), 8.36 (s, 1H), 7.76 (d, 1H), 7.74 (m, 5H), 7.59 (m, 2H), 6.28 (s, 2H), 5.89 (d, 1H); MS m/z : 601[M+H]

### Example 14. N-(4-((2-amino-3-iodopyridin-4-yl)oxy)-3,5-difluorophenyl)-1-(pyridin-2-yl)-5-(trifluoromethyl)-1H-pyrazole-4-carboxamide

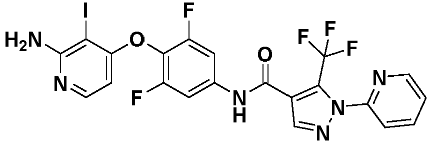

According to Example 13, 1-(pyridin-2-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (705 mg, yield: 55%) in a manner similar to Example 13.
¹H NMR (500 MHz, DMSO-d₆) δ 11.00 (s, 1H), 8.36 (s, 1H), 7.76 (d, 1H), 7.74 (m, 5H), 7.59 (m, 2H), 6.28 (s, 2H), 5.89 (d, 1H); MS m/z : 601[M+H]

### Example 15. N-(4-((2-amino-3-iodopyridin-4-yl)oxy)-3,5-difluorophenyl)-1-(3-fluoropyridin-2-yl)-5-(trifluoromethyl)-1H-pyrazole-4-carboxamide

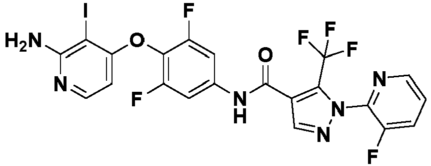

According to Example 13, 1-(3-fluoropyridin-2-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (700 mg, yield: 53%) in a manner similar to Example 13.
¹H NMR (500 MHz, DMSO-d₆) δ 11.05 (s, 1H), 8.56 (d, 1H), 8.50 (s, 1H), 8.23 (t, 1H), 7.88 (m, 1H), 7.76 (d, 1H), 7.68 (m, 2H), 6.29 (s, 2H), 5.89 (d, 1H); MS m/z : 621[M+H]

### Example 16. N-(4-((2-amino-3-iodopyridin-4-yl)oxy)-3,5-difluorophenyl)-1-(3-chloropyridin-2-yl)-5-(trifluoromethyl)-1H-pyrazole-4-carboxamide

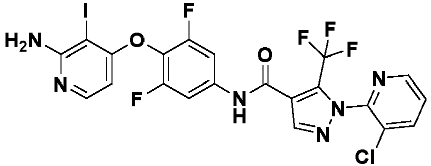

According to Example 13, 1-(3-chloropyridin-2-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (710 mg, yield: 58%) in a manner similar to Example 13.
¹H NMR (500 MHz, DMSO-d₆) δ 11.02 (s, 1H), 8.67 (d, 1H), 8.51 (s, 1H), 8.41 (d, 1H), 7.83 (m, 1H), 7.76 (d, 1H), 7.68 (m, 2H), 6.29 (s, 2H), 5.89 (d, 1H); MS m/z : 637[M+H]

### Example 17. N-(4-((2-amino-3-iodopyridin-4-yl)oxy)-3,5-difluorophenyl)-1-(pyridazin-3-yl)-5-(trifluoromethyl)-1H-pyrazole-4-carboxamide

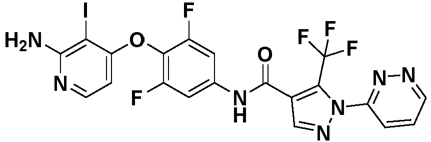

According to Example 13, 1-(pyridazin-3-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (690 mg, yield: 59%) in a manner similar to Example 13.
¹H NMR (500 MHz, DMSO-d₆) δ 11.10 (s, 1H), 9.47 (d, 1H), 8.52 (s, 1H), 8.26 (d, 1H), 8.11 (m, 1H), 7.76 (d, 1H), 7.66 (d, 2H), 6.29 (s, 2H), 5.90 (d, 1H); MS m/z : 604[M+H]

### Example 18. N-(4-((2-amino-3-iodopyridin-4-yl)oxy)-3,5-difluorophenyl)-1-(pyrimidin-2-yl)-5-(trifluoromethyl)-1H-pyrazole-4-carboxamide

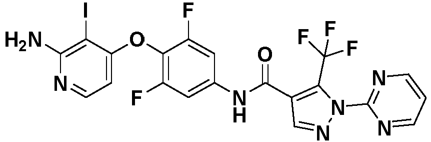

According to Example 13, 1-(pyrimidin-2-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (580 mg, yield: 57%) in a manner similar to Example 13.
¹H NMR (500 MHz, DMSO-d₆) δ 11.08 (s, 1H), 9.10 (d, 1H), 8.44 (s, 1H), 7.81 (t, 1H), 7.76 (d, 1H), 7.66 (d, 2H), 6.29 (s, 2H), 5.90 (d, 1H); MS m/z : 604[M+H]

### Example 19. N-(4-((2-amino-3-(tetrahydro-2H-pyran-4-yl)pyridin-4-yl)oxy)-3,5-difluorophenyl)-1-phenyl-5-(trifluoromethyl)-1H-pyrazole-4-carboxamide

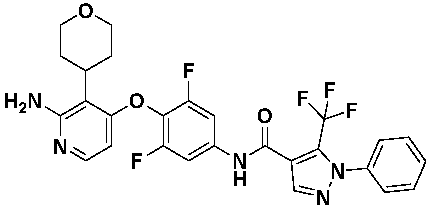

Hereinafter, a compound of Example 19 was prepared according to Scheme 6 below.

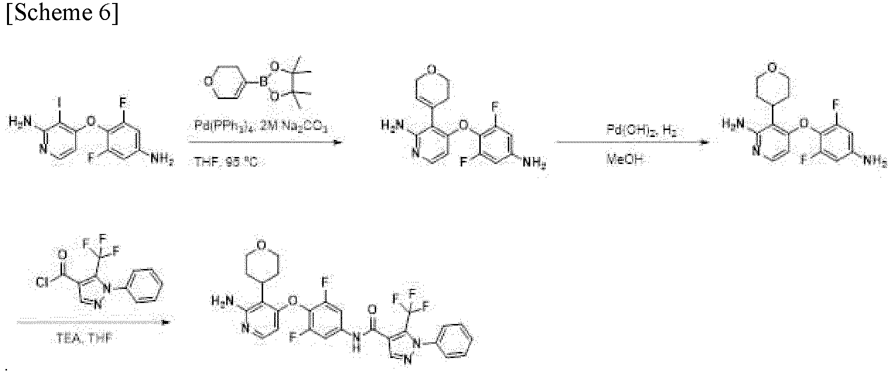

### Step 1) 4-(4-amino-2,6-difluorophenoxy)-3-(3,6-dihydro-2H-pyran-4-yl)pyridin-2-amine

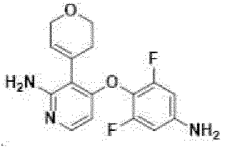

4-(4-amino-2,6-difluorophenoxy)-3-iodopyridin-2-amine (1 g, 2.75 mmol) was dissolved in 20 ml of 1,4-dioxane, 2-(3,6-dihydro-2H-pyran-4-yl)-4,4,5,5-tetramethyl-1,3,2-dioxaborolane (694 mg, 3.30 mmol), Pd(PPh₃)₄ (318 mg, 0.27 mmol), and 2 M sodium acetate (4.1 ml, 8.26 mmol) were added, and the resulting mixture was stirred at 95 °C for 12 h. The reaction mixture was cooled to room temperature, filtered through a Celite filter, and the filtrate was concentrated. The residue was purified by column chromatography to obtain the title compound (800 mg, yield: 91%).
MS m/z : 320[M+H]

### Step 2) 4-(4-amino-2,6-difluorophenoxy)-3-(tetrahydro-2H-pyran-4-yl)pyridin-2-amine

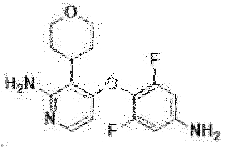

The compound 4-(4-amino-2,6-difluorophenoxy)-3-(3,6-dihydro-2H-pyran-4-yl)pyridin-2-amine (800 mg, 2.51 mmol) obtained in Step 1 was dissolved in 20 ml of methanol, Pd(OH)₂ (2.46 g, 17.54 mmol) was added, and the resulting mixture was stirred under hydrogen gas for 12 h. The reaction solution was filtered through a Celite filter, the filtrate was concentrated, and the residue was purified by column chromatography to obtain the title compound (400 mg, yield: 50%).
MS m/z : 322[M+H]

### Step 3) N-(4-((2-amino-3-(tetrahydro-2H-pyran-4-yl)pyridin-4-yl)oxy)-3,5-difluorophenyl)-1-phenyl-5-(trifluoromethyl)-1H-pyrazole-4-carboxamide

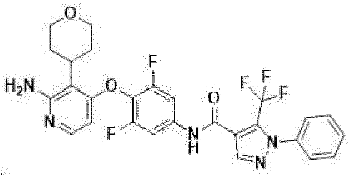

According to Example 1, the compound 4-(4-amino-2,6-difluorophenoxy)-3-(tetrahydro-2H-pyran-4-yl)pyridin-2-amine obtained in Step 2 was used instead of 4-(4-amino-2,6-difluorophenoxy)-3-chloropyridin-2-amine to obtain the title compound (18 mg, yield: 34%) in a manner similar to Example 1.
¹H NMR (500 MHz, DMSO-d₆) δ 10.97 (s, 1H), 8.36 (d, 1H), 7.67 (m, 6H), 7.57 (m, 2H), 5.99 (brs, 2H), 5.86 (d, 1H), 3.94 (m, 2H), 3.48 (t, 2H), 3.09 (m, 1H), 2.33 (m, 2H), 1.52 (m, 2H); MS m/z : 560[M+H]

### Example 20. N-(4-((2-amino-3-(tetrahydro-2H-pyran-4-yl)pyridin-4-yl)oxy)-3,5-difluorophenyl)-1-(3-fluoropyridin-2-yl)-5-(trifluoromethyl)-1H-pyrazole-4-carboxamide

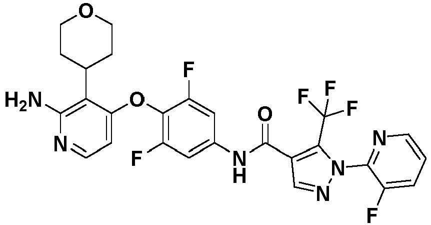

According to Example 19, 1-(3-fluoropyridin-2-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (14 mg, yield: 26%) in a manner similar to Example 19.
¹H NMR (500 MHz, DMSO-d₆) δ 11.03 (s, 1H), 8.56 (d, 1H), 8.51 (s, 1H), 8.23 (t, 1H), 7.88 (m, 1H), 7.66 (m, 3H), 5.99 (brs, 2H), 5.86 (d, 1H), 3.93 (m, 2H), 3.48 (t, 2H), 3.07 (m, 1H), 2.31 (m, 2H), 1.53 (m, 2H); MS m/z : 579[M+H]

### Example 21. N-(4-((2-amino-3-(tetrahydro-2H-pyran-4-yl)pyridin-4-yl)oxy)-3,5-difluorophenyl)-1-(3-chloropyridin-2-yl)-5-(trifluoromethyl)-1H-pyrazole-4-carboxamide

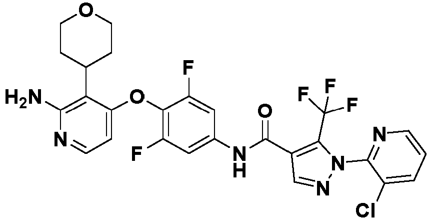

According to Example 19, 1-(3-chloropyridin-2-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (23 mg, yield: 41%) in a manner similar to Example 19.
¹H NMR (500 MHz, DMSO-d₆) δ 10.99 (s, 1H), 8.68 (d, 1H), 8.52 (s, 1H), 8.42 (m, 1H), 7.84 (m, 1H), 7.67 (m, 3H), 5.99 (brs, 2H), 5.86 (d, 1H), 3.94 (m, 2H), 3.46 (t, 2H), 3.07 (m, 1H), 2.33 (m, 2H), 1.52 (m, 2H); MS m/z : 595[M+H]

### Example 22. N-(4-((2-amino-3-(tetrahydro-2H-pyran-4-yl)pyridin-4-yl)oxy)-3,5-difluorophenyl)-1-(pyrimidin-2-yl)-5-(trifluoromethyl)-1H-pyrazole-4-carboxamide

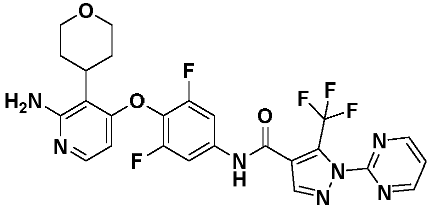

According to Example 19, 1-(pyrimidin-2-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (13 mg, yield: 25%) in a manner similar to Example 19.
¹H NMR (500 MHz, DMSO-d₆) δ 11.05 (s, 1H), 9.10 (d, 1H), 8.44 (s, 1H), 7.82 (m, 1H), 7.66 (m, 3H), 5.99 (brs, 2H), 5.85 (d, 1H), 3.94 (m, 2H), 3.45 (t, 2H), 3.09 (m, 1H), 2.31 (m, 2H), 1.50 (m, 2H); MS m/z : 562[M+H]

### Example 23. N-(4-((2-amino-3-(tetrahydro-2H-pyran-4-yl)pyridin-4-yl)oxy)-3,5-difluorophenyl)-1-(pyridazin-3-yl)-5-(trifluoromethyl)-1H-pyrazole-4-carboxamide

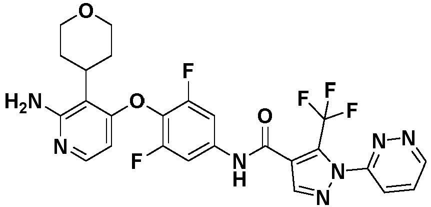

According to Example 19, 1-(pyridazin-3-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (12 mg, yield: 24%) in a manner similar to Example 19.
¹H NMR (500 MHz, DMSO-d₆) δ 11.10 (s, 1H), 9.47 (d, 1H), 8.55 (m, 1H), 8.26 (m, 1H), 8.12 (m, 1H), 7.75 (m, 1H), 7.66 (m, 3H), 5.99 (brs, 2H), 5.86 (d, 1H), 3.94 (m, 2H), 3.46 (t, 2H), 3.07 (m, 1H), 2.33 (m, 2H), 1.53 (m, 2H); MS m/z : 562[M+H]

### Example 24. N-(4-((2-amino-3-(tetrahydro-2H-pyran-4-yl)pyridin-4-yl)oxy)-3,5-difluorophenyl)-1-(pyridin-2-yl)-5-(trifluoromethyl)-1H-pyrazole-4-carboxamide

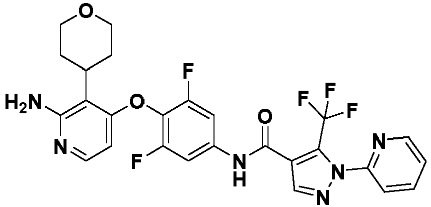

According to Example 19, 1-(pyridin-2-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (8 mg, yield: 14%) in a manner similar to Example 19.
¹H NMR (500 MHz, DMSO-d₆) δ 11.03 (s, 1H), 8.64 (m, 1H), 8.40 (m, 1H), 8.22 (m, 2H), 7.85 (d, 1H), 7.66 (m, 4H), 5.99 (brs, 2H), 5.86 (d, 1H), 3.94 (m, 2H), 3.48 (t, 2H), 3.07 (m, 1H), 2.33 (m, 2H), 1.52 (m, 2H); MS m/z : 561[M+H]

### Example 25. N-(4-((2-amino-3-ethynylpyridin-4-yl)oxy)-3,5-difluorophenyl)-1-phenyl-5-(trifluoromethyl)-1H-pyrazole-4-carboxamide

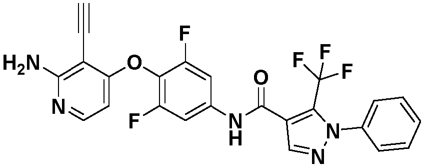

Hereinafter, a compound of Example 25 was prepared according to Scheme 7 below.

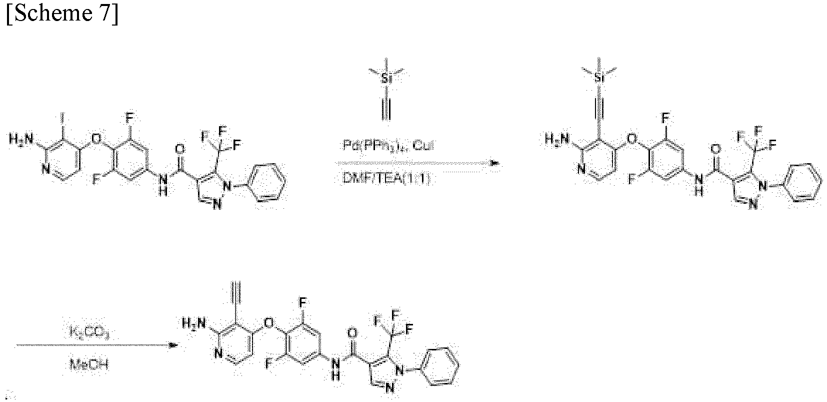

### Step 1) N-(4-((2-amino-3-((trimethylsilyl)ethynyl)pyridin-4-yl)oxy)-3,5-difluorophenyl)-1-phenyl-5-(trifluoromethyl)-1H-pyrazole-4-carboxamide

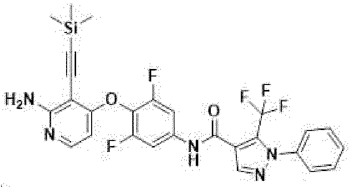

N-(4-((2-amino-3-iodopyridin-4-yl)oxy)-3,5-difluorophenyl)-1-phenyl-5-(trifluoromethyl)-1H-pyrazole-4-carboxamide (70 mg, 0.12 mmol) was dissolved in 1.5 ml of a DMF/TEA (1:1) solution, ethynyltrimethylsilane (32 µl, 0.24 mmol), Pd(PPh₃)₄ (13 mg, 0.01 mmol), and CuI (4.4 mg, 0.02 mmol) were added, and the resulting mixture was stirred at room temperature for four hours. The reaction solution was diluted with water, extracted three times with EA, and concentrated. The residue was purified by column chromatography to obtain the title compound (30 mg, yield: 45%).
MS m/z : 572[M+H]

### Step 2) N-(4-((2-amino-3-ethynylpyridin-4-yl)oxy)-3,5-difluorophenyl)-1-phenyl-5-(trifluoromethyl)-1H-pyrazole-4-carboxamide

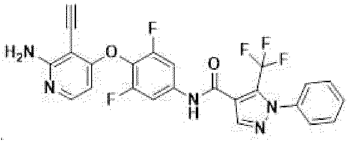

The compound N-(4-((2-amino-3-((trimethylsilyl)ethynyl)pyridin-4-yl)oxy)-3,5-difluorophenyl)-1-phenyl-5-(trifluoromethyl)-1H-pyrazole-4-carboxamide (40 mg, 0.07 mmol) obtained in Step 1 was dissolved in 4 ml of methanol, K₂CO₃ (40 mg, 0.28 mmol) was added, and the resulting mixture was stirred at room temperature for four hours. The reaction solution was diluted with water, extracted three times with EA, and concentrated. The residue was purified by column chromatography to obtain the title compound (21 mg, yield: 57%).
¹H NMR (500 MHz, DMSO-d₆) δ 11.00 (s, 1H), 8.35 (s, 1H), 7.77 (d, 1H), 7.66 (m, 5H), 7.57 (m, 2H), 6.39 (s, 2H), 5.96 (d, 1H), 4.64 (s, 1H) ; MS m/z : 500[M+H]

### Example 26. N-(4-((2-amino-3-ethynylpyridin-4-yl)oxy)-3,5-difluorophenyl)-1-(3-fluoropyridin-2-yl)-5-(trifluoromethyl)-1H-pyrazole-4-carboxamide

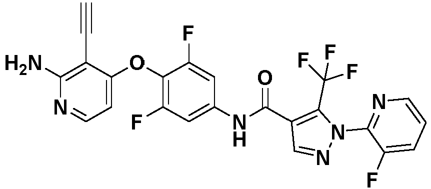

According to Example 25, N-(4-((2-amino-3-iodopyridin-4-yl)oxy)-3,5-difluorophenyl)-1-(3-fluoropyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (8 mg, yield: 46%) in a manner similar to Example 25.
¹H NMR (500 MHz, DMSO-d₆) δ 11.05 (s, 1H), 8.56 (d, 1H), 8.50 (s, 1H), 8.23 (t, 1H), 7.87 (m, 1H), 7.83 (d, 1H), 7.66 (m, 2H), 6.40 (s, 2H), 5.96 (d, 1H), 4.65 (s, 1H); MS m/z : 519[M+H]

### Example 27. N-(4-((2-amino-3-ethynylpyridin-4-yl)oxy)-3,5-difluorophenyl)-1-(3-chloropyridin-2-yl)-5-(trifluoromethyl)-1H-pyrazole-4-carboxamide

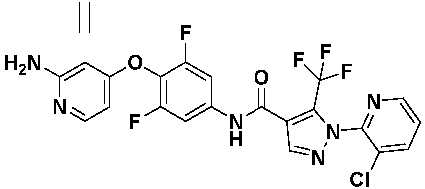

According to Example 25, N-(4-((2-amino-3-iodopyridin-4-yl)oxy)-3,5-difluorophenyl)-1-(3-chloropyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (20 mg, yield: 57%) in a manner similar to Example 25.
¹H NMR (500 MHz, DMSO-d6) δ 11.03 (s, 1H), 8.67 (d, 1H), 8.50 (s, 1H), 8.41 (d, 1H), 7.83 (m, 2H), 8.41 (m, 2H), 6.39 (s, 2H), 5.96 (d, 1H), 4.64 (s, 1H) ; MS m/z : 535[M+H]

### Example 28. N-(4-((2-amino-3-ethynylpyridin-4-yl)oxy)-3,5-difluorophenyl)-1-(pyrimidin-2-yl)-5-(trifluoromethyl)-1H-pyrazole-4-carboxamide

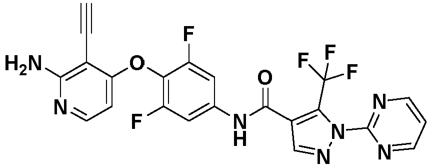

According to Example 25, N-(4-((2-amino-3-iodopyridin-4-yl)oxy)-3,5-difluorophenyl)-1-(pyrimidin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (8 mg, yield: 31%) in a manner similar to Example 25.
¹H NMR (500 MHz, DMSO-d₆) δ 11.08 (s, 1H), 9.10 (d, 1H), 8.36 (s, 1H), 7.82 (m, 2H), 7.66 (m, 2H), 6.39 (s, 2H), 5.97 (d, 1H), 4.64 (s, 1H); MS m/z : 502[M+H]

### Example 29. N-(4-((2-amino-3-ethynylpyridin-4-yl)oxy)-3,5-difluorophenyl)-1-(pyridin-2-yl)-5-(trifluoromethyl)-1H-pyrazole-4-carboxamide

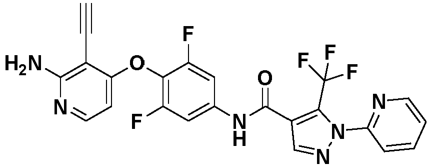

According to Example 25, N-(4-((2-amino-3-iodopyridin-4-yl)oxy)-3,5-difluorophenyl)-1-(pyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (3 mg, yield: 34%) in a manner similar to Example 25.
¹H NMR (500 MHz, DMSO-d₆) δ 11.04 (s, 1H), 8.63 (s, 1H), 8.39 (s, 1H), 8.16 (t, 1H), 7.84 (m, 2H), 7.67 (m, 3H), 6.39 (s, 2H), 5.96 (d, 1H), 4.64 (s, 1H) ; MS m/z : 501[M+H]

### Example 30. N-(4-((2-amino-3-ethynylpyridin-4-yl)oxy)-3,5-difluorophenyl)-1-(pyridazin-3-yl)-5-(trifluoromethyl)-1H-pyrazole-4-carboxamide

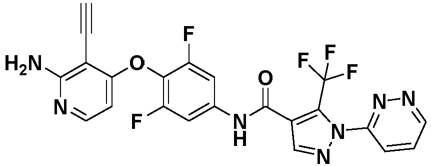

According to Example 25, N-(4-((2-amino-3-iodopyridin-4-yl)oxy)-3,5-difluorophenyl)-1-(pyridazin-3-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (17 mg, yield: 65%) in a manner similar to Example 25.
¹H NMR (500 MHz, DMSO-d₆) δ 11.10 (s, 1H), 9.47 (d, 1H), 8.52 (s, 1H), 8.24 (d, 1H), 8.11 (t, 1H), 7.83 (d, 1H), 7.66 (m, 2H), 6.39 (s, 2H), 5.97 (d, 1H), 4.64 (s, 1H) ; MS m/z : 502[M+H]

### Example 31. N-(4-((2-amino-3-cyanopyridin-4-yl)oxy)-3,5-difluorophenyl)-1-phenyl-5-(trifluoromethyl)-1H-pyrazole-4-carboxamide

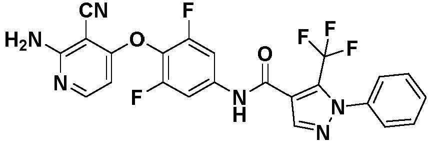

Hereinafter, a compound of Example 31 was prepared according to Scheme 8 below.

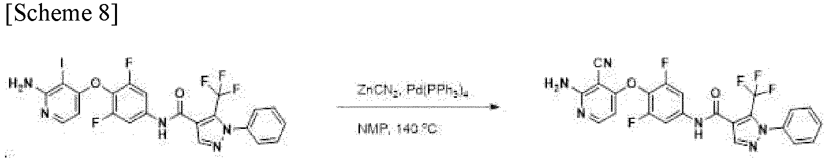

N-(4-((2-amino-3-iodopyridin-4-yl)oxy)-3,5-difluorophenyl)-1-phenyl-5-(trifluoromethyl)-1H-pyrazole-4-carboxamide (50 mg, 0.08 mmol) was dissolved in 1 ml of N-methyl-2-pyrrolidone, Pd(PPh₃)₄ (20 mg, 0.02 mmol) and ZnCN₂ (10 mg, 0.08 mmol) were added, and the resulting mixture was stirred at 140 °C for 12 h. The reaction solution was diluted with water, extracted three times with EA, and concentrated. The residue was purified by column chromatography to obtain the title compound (9 mg, yield: 22%).
¹H NMR (500 MHz, DMSO-d₆) δ 11.10 (s, 1H), 8.36 (s, 1H), 8.06 (d, 1H), 7.71 (d, 2H), 7.56 (m, 5H), 7.18 (br s, 2H), 6.09 (d, 1H); MS m/z : 501[M+H]

### Example 32. N-(4-((2-amino-3-cyanopyridin-4-yl)oxy)-3,5-difluorophenyl)-1-(pyridin-2-yl)-5-(trifluoromethyl)-1H-pyrazole-4-carboxamide

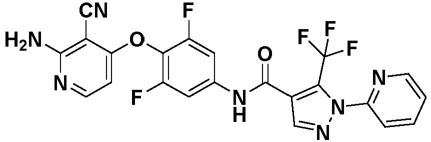

According to Example 31, N-(4-((2-amino-3-iodopyridin-4-yl)oxy)-3,5-difluorophenyl)-1-(pyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (20 mg, yield: 48%) in a manner similar to Example 31.
¹H NMR (500 MHz, DMSO-d6) δ 11.12 (s, 1H), 8.63 (d, 1H), 8.39 (s, 1H), 8.17 (t, 1H), 8.06 (d, 1H), 7.85 (d, 1H), 7.66 (m, 3H), 7.18 (brs, 2H), 6.09 (d, 1H); MS m/z : 502[M+H]

### Example 33. N-(4-((2-amino-3-cyanopyridin-4-yl)oxy)-3,5-difluorophenyl)-1-(3-fluoropyridin-2-yl)-5-(trifluoromethyl)-1H-pyrazole-4-carboxamide

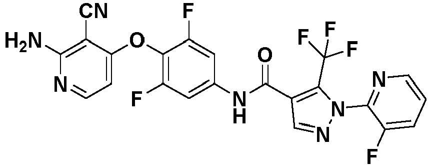

According to Example 31, N-(4-((2-amino-3-iodopyridin-4-yl)oxy)-3,5-difluorophenyl)-1-(3-fluoropyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (20 mg, yield: 48%) in a manner similar to Example 31.
¹H NMR (500 MHz, DMSO-d₆) 11.13 (s, 1H), 8.55 (s, 1H), 8.50 (s, 1H), 8.23 (t, 1H), 8.06 (d, 1H), 7.87 (m, 1H), 7.71 (m, 2H), 7.18 (brs, 2H), 6.09 (d, 1H); MS m/z : 520[M+H]

### Example 34. N-(4-((2-amino-3-cyanopyridin-4-yl)oxy)-3,5-difluorophenyl)-1-(3-chloropyridin-2-yl)-5-(trifluoromethyl)-1H-pyrazole-4-carboxamide

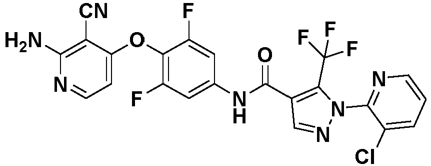

According to Example 31, N-(4-((2-amino-3-iodopyridin-4-yl)oxy)-3,5-difluorophenyl)-1-(3-chloropyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (20 mg, yield: 47%) in a manner similar to Example 31.
¹H NMR (500 MHz, DMSO-d₆) δ 11.11 (s, 1H), 8.67 (d, 1H), 8.52 (s, 1H), 8.41 (d, 1H), 8.06 (d, 1H), 7.83 (m, 1H), 7.71 (m, 2H), 7.18 (brs, H), 6.09 (d, 1H); MS m/z : 536[M+H]

### Example 35. N-(4-((2-amino-3-cyanopyridin-4-yl)oxy)-3,5-difluorophenyl)-1-(pyrimidin-2-yl)-5-(trifluoromethyl)-1H-pyrazole-4-carboxamide

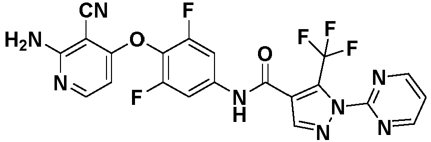

According to Example 31, N-(4-((2-amino-3-iodopyridin-4-yl)oxy)-3,5-difluorophenyl)-1-(pyrimidin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (20 mg, yield: 48%) in a manner similar to Example 31.
¹H NMR (500 MHz, DMSO-d₆) δ 11.17 (s, 1H), 9.10 (d, 1H), 8.44 (s, 1H), 8.06 (d, 1H), 7.83 (m, 1H), 7.71 (m, 2H), 7.18 (brs, H), 6.09 (d, 1H); MS m/z : 503[M+H]

### Example 36. N-(4-((2-amino-3-cyanopyridin-4-yl)oxy)-3,5-difluorophenyl)-1-(pyridazin-3-yl)-5-(trifluoromethyl)-1H-pyrazole-4-carboxamide

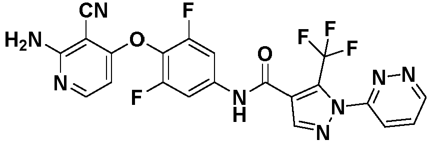

According to Example 31, N-(4-((2-amino-3-iodopyridin-4-yl)oxy)-3,5-difluorophenyl)-1-(pyridazin-3-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (20 mg, yield: 48%) in a manner similar to Example 31.
¹H NMR (500 MHz, DMSO-d₆) δ 11.22 (s, 1H), 9.47 (d, 1H), 8.53 (s, 1H), 8.26 (d, 1H), 8.12 (m, 1H), 8.07 (d, 1H), 7.72 (m, 2H), 7.19 (brs, H), 6.09 (d, 1H); MS m/z : 503[M+H]

### Example 37. N-(4-((2-amino-3-cyclopropylpyridin-4-yl)oxy)-3,5-difluorophenyl)-1-phenyl-5-(trifluoromethyl)-1H-pyrazole-4-carboxamide

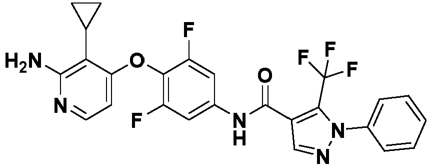

According to Example 19, cyclopropylboronic acid was used instead of 2-(3,6-dihydro-2H-pyran-4-yl)-4,4,5,5-tetramethyl-1,3,2-dioxaborolane to obtain the title compound (15 mg, yield: 18%) in a manner similar to Example 19.
¹H NMR (500 MHz, DMSO-d₆) δ 10.91 (s, 1H), 8.31 (s, 1H), 7.59 (m, 8H), 5.87 (brs, H), 5.78 (d, 1H), 1.40 (m, 1H), 0.96 (m, 2H), 0.56 (m, 2H); MS m/z : 516[M+H]

### Example 38. N-(4-((2-amino-3-cyclopropylpyridin-4-yl)oxy)-3,5-difluorophenyl)-1-(3-fluoropyridin-2-yl)-5-(trifluoromethyl)-1H-pyrazole-4-carboxamide

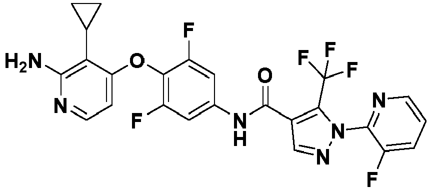

According to Example 37, N-(4-((2-amino-3-iodopyridin-4-yl)oxy)-3,5-difluorophenyl)-1-(3-fluoropyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (15 mg, yield: 17%) in a manner similar to Example 37.
¹H NMR (500 MHz, DMSO-d₆) δ 11.01 (s, 1H), 8.56 (d, 1H), 8.50 (s, 1H), 8.23 (t, 1H), 7.88 (m, 1H), 7.66 (m, 3H), 5.91 (brs, 2H), 5.82 (d, 1H), 1.46 (m, 1H), 1.02 (m, 2H), 0.61 (m, 2H) ; MS m/z : 535[M+H]

### Example 39. N-(4-((2-amino-3-cyclopropylpyridin-4-yl)oxy)-3,5-difluorophenyl)-1-(3-chloropyridin-2-yl)-5-(trifluoromethyl)-1H-pyrazole-4-carboxamide

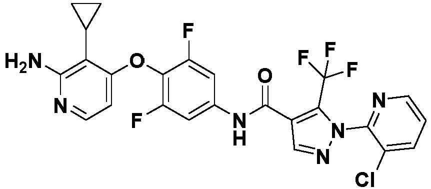

According to Example 37, N-(4-((2-amino-3-iodopyridin-4-yl)oxy)-3,5-difluorophenyl)-1-(3-chloropyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (15 mg, yield: 17%) in a manner similar to Example 37.
¹H NMR (500 MHz, DMSO-d₆) δ 10.97 (s, 1H), 8.67 (d, 1H), 8.50 (s, 1H), 8.41 (d, 1H), 7.83 (m, 1H), 7.66 (m, 3H), 5.91 (brs, 2H), 5.82 (d, 1H), 1.46 (m, 1H), 1.02 (m, 2H), 0.61 (m, 2H) ; MS m/z : 551[M+H]

### Example 40. N-(4-((2-amino-3-cyclopropylpyridin-4-yl)oxy)-3,5-difluorophenyl)-1-(pyridin-2-yl)-5-(trifluoromethyl)-1H-pyrazole-4-carboxamide

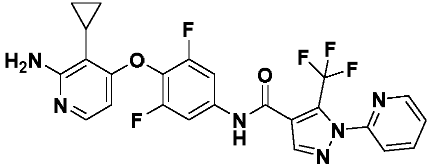

According to Example 37, N-(4-((2-amino-3-iodopyridin-4-yl)oxy)-3,5-difluorophenyl)-1-(pyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (30 mg, yield: 35%) in a manner similar to Example 37.
¹H NMR (500 MHz, DMSO-d₆) δ 8 11.00 (s, 1H), 8.63 (d, 1H), 8.39 (s, 1H), 8.16 (m, 1H), 7.85 (d, 1H), 7.66 (m, 4H), 5.91 (brs, 2H), 5.82 (d, 1H), 1.46 (m, 1H), 1.02 (m, 2H), 0.61 (m, 2H) ; MS m/z : 517[M+H]

### Example 41. N-(4-((2-amino-3-cyclopropylpyridin-4-yl)oxy)-3,5-difluorophenyl)-1-(pyrimidin-2-yl)-5-(trifluoromethyl)-1H-pyrazole-4-carboxamide

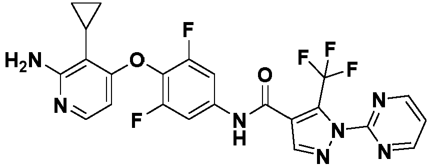

According to Example 37, N-(4-((2-amino-3-iodopyridin-4-yl)oxy)-3,5-difluorophenyl)-1-(pyrimidin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (10 mg, yield: 12%) in a manner similar to Example 37.
¹H NMR (500 MHz, DMSO-d₆) δ 11.30 (s, 1H), 9.10 (d, 1H), 8.43 (s, 1H), 7.83 (m, 1H), 7.66 (m, 3H), 5.91 (brs, 2H), 5.82 (d, 1H), 1.46 (m, 1H), 1.02 (m, 2H), 0.61 (m, 2H) ; MS m/z : 518[M+H]

### Example 42. N-(4-((2-amino-3-cyclopropylpyridin-4-yl)oxy)-3,5-difluorophenyl)-1-(pyridazin-3-yl)-5-(trifluoromethyl)-1H-pyrazole-4-carboxamide

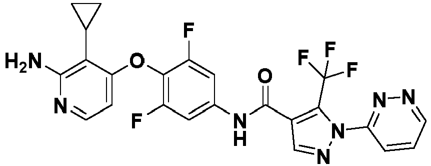

According to Example 37, N-(4-((2-amino-3-iodopyridin-4-yl)oxy)-3,5-difluorophenyl)-1-(pyridazin-3-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (15 mg, yield: 17%) in a manner similar to Example 37.
¹H NMR (500 MHz, DMSO-d₆) δ 11.07 (s, 1H), 9.47 (d, 1H), 8.52 (s, 1H), 8.25 (d, 1H), 8.11 (m, 1H), 7.69 (m, 3H), 5.91 (brs, 2H), 5.82 (d, 1H), 1.46 (m, 1H), 1.02 (m, 2H), 0.61 (m, 2H) ; MS m/z : 518[M+H]

### Example 43. N-(4-((2-amino-3-isopropylpyridin-4-yl)oxy)-3,5-difluorophenyl)-1-phenyl-5-(trifluoromethyl)-1H-pyrazole-4-carboxamide

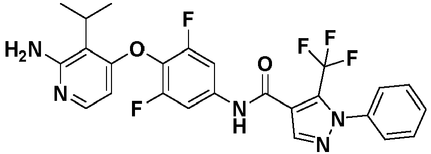

According to Example 19, 4,4,5,5-tetramethyl-2-(prop-1-en-2-yl)-1,3,2-dioxaborolane was used instead of 2-(3,6-dihydro-2H-pyran-4-yl)-4,4,5,5-tetramethyl-1,3,2-dioxaborolane to obtain the title compound (13 mg, yield: 25%) in a manner similar to Example 19.
¹H NMR (500 MHz, DMSO-d₆) δ 10.95 (s, 1H), 8.36 (d, 1H), 7.65 (m, 5H), 7.56 (m, 2H), 5.85 (brs, 3H), 3.23 (m, 1H), 1.35 (d, 6H); MS m/z : 518[M+H]

### Example 44. N-(4-((2-amino-3-isopropylpyridin-4-yl)oxy)-3,5-difluorophenyl)-1-(3-chloropyridin-2-yl)-5-(trifluoromethyl)-1H-pyrazole-4-carboxamide

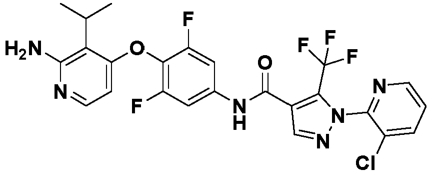

According to Example 43, N-(4-((2-amino-3-iodopyridin-4-yl)oxy)-3,5-difluorophenyl)-1-(3-chloropyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (6 mg, yield: 20%) in a manner similar to Example 43.
¹H NMR (500 MHz, DMSO-d₆) δ 10.99 (s, 1H), 8.68 (d, 1H), 8.51 (s, 1H), 8.42 (d, 1H), 7.82 (m, 1H), 7.65 (m, 3H), 5.85 (brs, 3H), 3.22 (m, 1H), 1.34 (d, 6H); MS m/z : 553[M+H]

### Example 45. N-(4-((2-amino-3-isopropylpyridin-4-yl)oxy)-3,5-difluorophenyl)-1-(pyrimidin-2-yl)-5-(trifluoromethyl)-1H-pyrazole-4-carboxamide

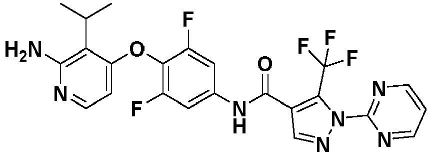

According to Example 43, N-(4-((2-amino-3-iodopyridin-4-yl)oxy)-3,5-difluorophenyl)-1-(pyrimidin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (2.3 mg, yield: 12%) in a manner similar to Example 43.
¹H NMR (500 MHz, DMSO-d₆) δ 11.05 (brs, 1H), 9.09 (m, 2H), 8.43 (s, 1H), 7.82 (m, 1H), 7.64 (m, 3H), 5.85 (brs, 3H), 3.23 (m, 1H), 1.35 (d, 6H); MS m/z : 520[M+H]

### Example 46. N-(4-((2-amino-3-isopropylpyridin-4-yl)oxy)-3,5-difluorophenyl)-1-(3-fluoropyridin-2-yl)-5-(trifluoromethyl)-1H-pyrazole-4-carboxamide

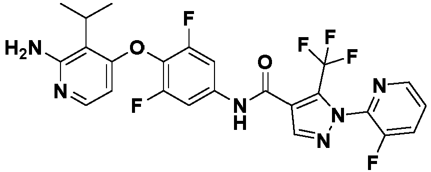

According to Example 43, N-(4-((2-amino-3-iodopyridin-4-yl)oxy)-3,5-difluorophenyl)-1-(3-fluoropyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (9 mg, yield: 22%) in a manner similar to Example 43.
¹H NMR (500 MHz, DMSO-d₆) 11.07 (s, 1H), 8.56 (m, 1H), 8.51 (m, 1H), 8.23 (m, 1H), 7.87 (m, 1H), 7.65 (m, 3H), 5.84 (brs, 3H), 3.23 (m, 1H), 1.34 (d, 6H); MS m/z : 537[M+H]

### Example 47. N-(4-((2-amino-3-(morpholinomethyl)pyridin-4-yl)oxy)-3,5-difluorophenyl)-1-(3-fluoropyridine-2-yl)-5-(trifluoromethyl)-1H-pyrazole-4-carboxamide

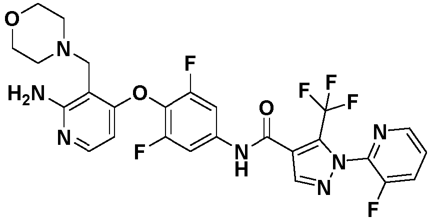

Hereinafter, a compound of Example 47 was prepared according to Scheme 9 below.

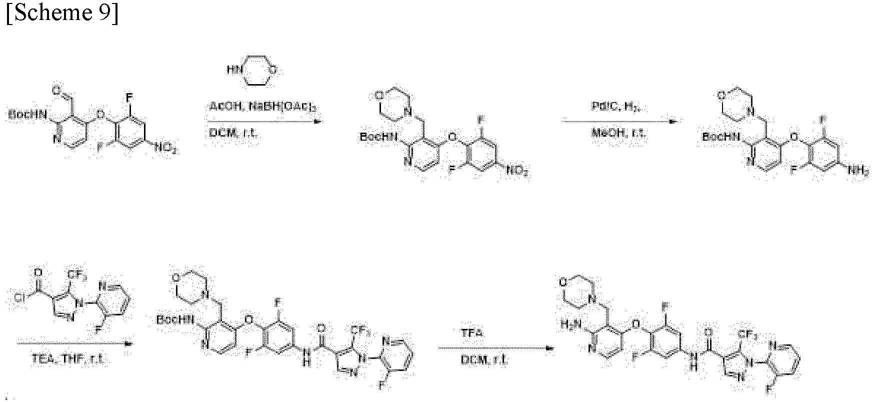

### Step 1) tert-Butyl (4-(2,6-difluoro-4-nitrophenoxy)-3-(morpholinomethyl)pyridin-2-yl)carbamate

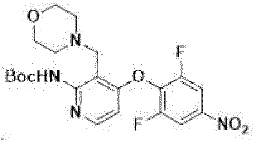

tert-Butyl (4-(2,6-difluoro-4-nitrophenoxy)-3-formylpyridin-2-yl)carbamate (1 g, 2.53 mmol) was dissolved in 50 ml of DCM, morpholine (264 µl, 3.04 mmol) and acetic acid (174 µl, 3.04 mmol) were added, and the resulting mixture was stirred at room temperature. After 30 minutes, sodium triacetoxyborohydride (1.07 g, 5.06 mmol) was added, and the resulting mixture was allowed to react at room temperature for 12 h. The reaction solution was diluted with a saturated aqueous NaHCO3 solution, extracted three times with DCM, and concentrated. The residue was purified by column chromatography to obtain the title compound (0.7 g, yield: 59%).
MS m/z : 467[M+H]

### Step 2) tert-Butyl (4-(4-amino-2,6-difluorophenoxy)-3-(morpholinomethyl)pyridin-2-yl)carbamate

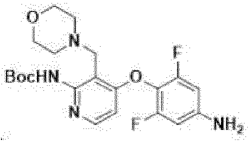

The compound tert-butyl (4-(2,6-difluoro-4-nitrophenoxy)-3-(morpholinomethyl)pyridin-2-yl)carbamate (600 mg, 1.29 mmol) obtained in Step 1 was dissolved in 15 ml of methanol, Pd/C (98 mg, 0.92 mmol) was added, and the resulting mixture was stirred for 12 h under hydrogen gas. The reaction solution was filtered through a Celite filter, the filtrate was concentrated, and the residue was purified by column chromatography to obtain the title compound (500 mg, yield: 57%).
MS m/z : 437[M+H]

### Step 3) tert-Butyl (4-(2,6-difluoro-4-(1-(3-fluoropyridin-2-yl)-5-(trifluoromethyl)-1H-pyrazole-4-carboxamido)phenoxy)-3-(morpholinomethyl)pyridin-2-yl)carbamate

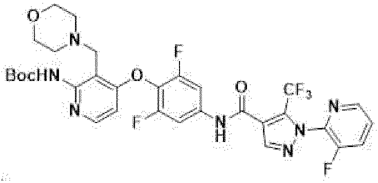

According to Example 1, tert-butyl (4-(4-amino-2,6-difluorophenoxy)-3-(morpholinomethyl)pyridin-2-yl)carbamate obtained in Step 2 was used instead of 4-(4-amino-2,6-difluorophenoxy)-3-chloropyridin-2-amine to obtain the title compound (80 mg, yield: 62%) in a manner similar to Example 1.
MS m/z : 694[M+H]

### Step 4) N-(4-((2-amino-3-(morpholinomethyl)pyridin-4-yl)oxy)-3,5-difluorophenyl)-1-(3-fluoropyridin-2-yl)-5-(trifluoromethyl)-1H-pyrazole-4-carboxamide

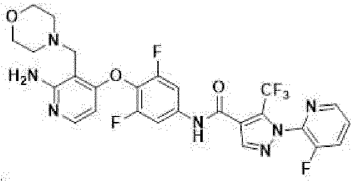

The compound tert-butyl (4-(2,6-difluoro-4-(1-(3-fluoropyridin-2-yl)-5-(trifluoromethyl)-1H-pyrazole-4-carboxamido)phenoxy)-3-(morpholinomethyl)pyridin-2-yl)carbamate (80 mg, 0.12 mmol) (80 mg, 0.12 mmol) obtained in Step 3 was dissolved in 3 ml of DCM, 3 ml of TFA was added, and the resulting mixture was stirred at room temperature for two hours. The reaction mixture was concentrated, and then the residue was diluted with EA and washed with a saturated aqueous NaHCO₃ solution. The organic layer was washed again with brine and concentrated, and the residue was purified by column chromatography to obtain the title compound (21 mg, yield: 31%).
¹H NMR (500 MHz, DMSO-d₆) δ 11.02 (s, 1H), 8.56 (d, 1H), 8.50 (s, 1H), 8.23 (t, 1H), 7.88 (brs, 1H), 7.77 (d, 1H), 7.65 (m, 2H), 6.21 (s, 2H), 5.90 (d, 1H), 3.59 (brs, 6H), 2.43 (s, 4H) ; MS m/z : 594[M+H]

### Example 48. N-(4-((2-amino-3-(morpholinomethyl)pyridin-4-yl)oxy)-3,5-difluorophenyl)-1-(pyrimidin-2-yl)-5-(trifluoromethyl)-1H-pyrazole-4-carboxamide

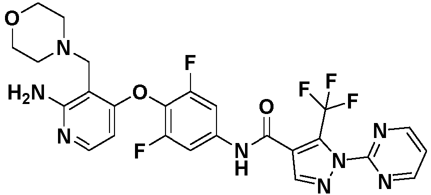

According to Example 47, 1-(pyrimidin-2-yl)-5-(trifluoromethyl)-1H-pyrazole-4-carbonyl chloride was used instead of 1-(3-fluoropyridin-2-yl)-5-(trifluoromethyl)-1H-pyrazole-4-carbonyl chloride to obtain the title compound (20 mg, yield: 29%) in a manner similar to Example 47.
¹H NMR (500 MHz, DMSO-d₆) δ 11.06 (s, 1H), 9.10 (d, 1H), 8.43 (s, 1H), 7.82 (t, 1H), 7.77 (d, 1H), 7.64 (d, 1H), 6.20 (s, 2H), 5.91 (d, 1H), 3.59 (s, 6H), 2.08 (s, 4H) ; MS m/z : 577[M+H]

### Example 49. N-(4-((2-amino-3-(morpholinomethyl)pyridin-4-yl)oxy)-3,5-difluorophenyl)-1-(3-chloropyridin-2-yl)-5-(trifluoromethyl)-1H-pyrazole-4-carboxamide

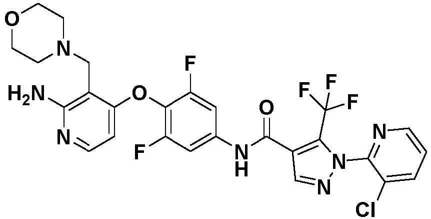

According to Example 47, 1-(3-chloropyridin-2-yl)-5-(trifluoromethyl)-1H-pyrazole-4-carbonyl chloride was used instead of 1-(3-fluoropyridin-2-yl)-5-(trifluoromethyl)-1H-pyrazole-4-carbonyl chloride to obtain the title compound (50 mg, yield: 58%) in a manner similar to Example 47.
¹H NMR (500 MHz, DMSO-d6) δ 10.99 (s, 1H), 8.67 (d, 1H), 8.51 (s, 1H), 8.41 (d, 1H), 7.82 (t, 1H), 7.77 (d, 1H), 7.66 (m, 2H), 6.20 (s, 2H), 5.90 (d, 1H), 3.59 (m, 6H), 2.44 (s, 4H) ; MS m/z : 610[M+H]

### Example 50. N-(4-((2-amino-3-(morpholinomethyl)pyridin-4-yl)oxy)-3,5-difluorophenyl)-1-phenyl-5-(trifluoromethyl)-1H-pyrazole-4-carboxamide

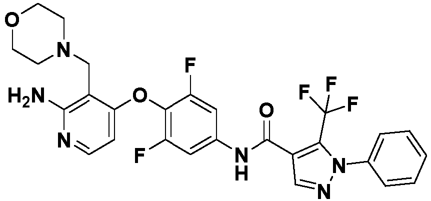

According to Example 47, 1-phenyl-5-(trifluoromethyl)-1H-pyrazole-4-carbonyl chloride was used instead of 1-(3-fluoropyridin-2-yl)-5-(trifluoromethyl)-1H-pyrazole-4-carbonyl chloride to obtain the title compound (30 mg, yield: 44%) in a manner similar to Example 47.
¹H NMR (500 MHz, DMSO-d₆) δ 10.96 (s, 1H), 8.35 (s, 1H), 7.77 (d, 1H), 7.63 (m, 5H), 7.57 (m, 2H), 6.20 (s, 2H), 5.90 (d, 1H), 3.60 (m, 6H), 2.43 (s, 4H) ; MS m/z : 575[M+H]

### Example 51. N-(4-((2-amino-3-(3-hydroxyprop-1-yn-1-yl)pyridin-4-yl)oxy)-3,5-difluorophenyl)-1-phenyl-5-(trifluoromethyl)-1H-pyrazole-4-carboxamide

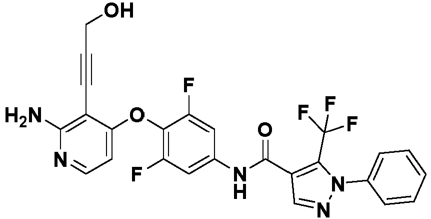

According to Example 25, prop-2-yn-1-ol was used instead of ethynyltrimethylsilane to obtain the title compound (30 mg, yield: 68%) in a manner similar to Example 25.
¹H NMR (500 MHz, DMSO-d₆) δ 10.97 (s, 1H), 8.34 (s, 1H), 7.80 (d, 1H), 7.62 (m, 6H), 6.04 (brs, 2H), 5.93 (d, 1H), 5.31 (t, 1H), 4.36 (d, 2H); MS m/z : 530[M+H]

### Example 52. N-(4-((2-amino-3-(3-hydroxyprop-1-yn-1-yl)pyridin-4-yl)oxy)-3,5-difluorophenyl)-1-(pyridin-2-yl)-5-(trifluoromethyl)-1H-pyrazole-4-carboxamide

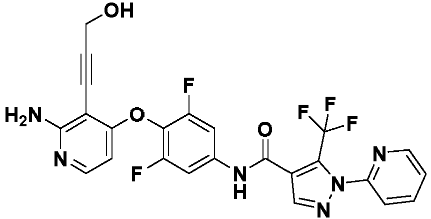

According to Example 51, N-(4-((2-amino-3-iodopyridin-4-yl)oxy)-3,5-difluorophenyl)-1-(pyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (30 mg, yield: 69%) in a manner similar to Example 51.
¹H NMR (500 MHz, DMSO-d₆) δ 11.03 (s, 1H), 8.63 (d, 1H), 8.39 (s, 1H), 8.14 (t, 1H), 7.85 (d, 1H), 7.79 (d, 1H), 7.63 (m, 3H), 6.41 (brs, 2H), 5.93 (d, 1H), 5.76 (s, 1H), 5.31 (t, 1H), 4.36 (d, 2H); MS m/z : 531[M+H]

### Example 53. N-(4-((2-amino-3-(3-hydroxyprop-1-yn-1-yl)pyridin-4-yl)oxy)-3,5-difluorophenyl)-1-(3-fluoropyridin-2-yl)-5-(trifluoromethyl)-1H-pyrazole-4-carboxamide

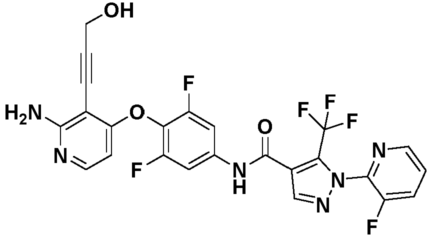

According to Example 51, N-(4-((2-amino-3-iodopyridin-4-yl)oxy)-3,5-difluorophenyl)-1-(3-fluoropyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (30 mg, yield: 66%) in a manner similar to Example 51.
¹H NMR (500 MHz, DMSO-d₆) δ 11.03 (s, 1H), 8.56 (d, 1H), 8.49 (s, 1H), 8.23 (t, 1H), 7.87 (t, 1H), 7.79 (d, 1H), 7.65 (d, 2H), 6.41 (brs, 2H), 5.93 (d, 1H), 5.31 (t, 1H), 4.36 (d, 2H); MS m/z : 549[M+H]

### Example 54. N-(4-((2-amino-3-(3-hydroxyprop-1-yn-1-yl)pyridin-4-yl)oxy)-3,5-difluorophenyl)-1-(3-chloropyridin-2-yl)-5-(trifluoromethyl)-1H-pyrazole-4-carboxamide

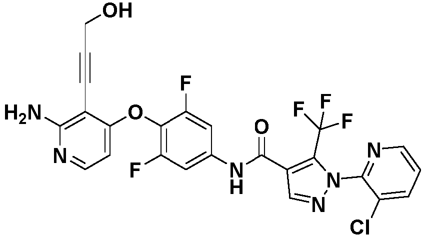

According to Example 51, N-(4-((2-amino-3-iodopyridin-4-yl)oxy)-3,5-difluorophenyl)-1-(3-chloropyridin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (30 mg, yield: 67%) in a manner similar to Example 51.
¹H NMR (500 MHz, DMSO-d₆) δ 11.00 (s, 1H), 8.67 (d, 1H), 8.50 (s, 1H), 8.41 (d, 1H), 7.83 (m, 2H), 7.65 (d, 2H), 6.41 (brs, 2H), 5.93 (d, 1H), 5.31 (t, 1H), 4.36 (d, 2H); MS m/z : 565[M+H]

### Example 55. N-(4-((2-amino-3-(3-hydroxyprop-1-yn-1-yl)pyridin-4-yl)oxy)-3,5-difluorophenyl)-1-(pyrimidin-2-yl)-5-(trifluoromethyl)-1H-pyrazole-4-carboxamide

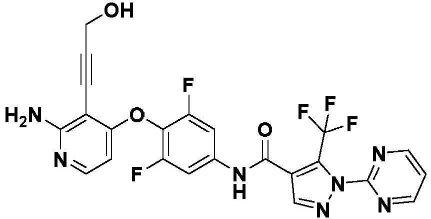

According to Example 51, N-(4-((2-amino-3-iodopyridin-4-yl)oxy)-3,5-difluorophenyl)-1-(pyrimidin-2-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (30 mg, yield: 68%) in a manner similar to Example 51.
¹H NMR (500 MHz, DMSO-d₆) δ 11.06 (s, 1H), 9.10 (d, 1H), 8.43 (s, 1H), 7.82 (m, 2H), 7.64 (d, 2H), 6.41 (brs, 2H), 5.93 (d, 1H), 5.31 (t, 1H), 4.36 (d, 2H); MS m/z : 532[M+H]

### Example 56. N-(4-((2-amino-3-(3-hydroxyprop-1-yn-1-yl)pyridin-4-yl)oxy)-3,5-difluorophenyl)-1-(pyridazin-3-yl)-5-(trifluoromethyl)-1H-pyrazole-4-carboxamide

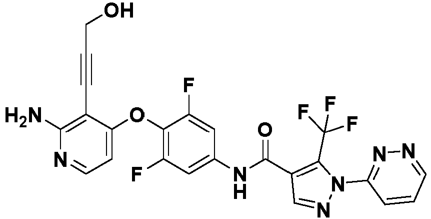

According to Example 51, N-(4-((2-amino-3-iodopyridin-4-yl)oxy)-3,5-difluorophenyl)-1-(pyridazin-3-yl)-5-(trifluoromethyl)-1H-pyrazole-4-carboxamide was used instead of N-(4-((2-amino-3-iodopyridin-4-yl)oxy)-3,5-difluorophenyl)-1-phenyl-5-(trifluoromethyl)-1H-pyrazole-4-carboxamide to obtain the title compound (30 mg, yield: 68%) in a manner similar to Example 51.
¹H NMR (500 MHz, DMSO-d₆) δ 11.09 (s, 1H), 9.47 (d, 1H), 8.52 (s, 1H), 8.25 (d, 1H), 8.11 (m, 1H), 7.81 (d, 2H), 7.67 (d, 1H), 6.41 (brs, 2H), 5.93 (d, 1H), 5.31 (t, 1H), 4.36 (d, 2H); MS m/z : 532[M+H]

### Example 57. N-(4-((2-amino-3-(3-hydroxybut-1-yn-1-yl)pyridin-4-yl)oxy)-3,5-difluorophenyl)-1-(3-fluoropyridin-2-yl)-5-(trifluoromethyl)-1H-pyrazole-4-carboxamide

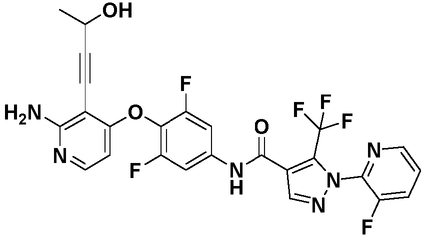

According to Example 53, but-3-yn-2-ol was used instead of prop-2-yn-1-ol to obtain the title compound (25 mg, yield: 43%) in a manner similar to Example 53.
¹H NMR (500 MHz, DMSO-d₆) δ 11.04 (s, 1H), 8.52 (d, 1H), 8.45 (s, 1H), 8.25 (t, 1H), 7.81 (2m, 2H), 7.67 (d, 2H), 6.35 (brs, 2H), 5.93 (d, 1H), 5.45 (d, 1H), 4.75 (m, 1H), 1.35 (d, 3H); MS m/z : 563 [M+H]

### Example 58. N-(4-((2-amino-3-(3-hydroxy-3-methylbut-1-yn-1-yl)pyridin-4-yl)oxy)-3,5-difluorophenyl)-1-(3-fluoropyridin-2-yl)-5-(trifluoromethyl)-1H-pyrazole-4-carboxamide

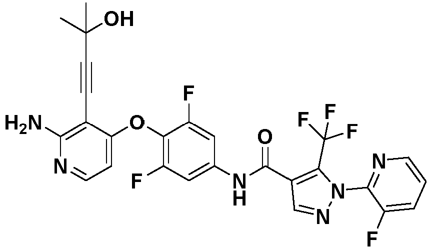

According to Example 53, 2-methylbut-3-yn-2-ol was used instead of prop-2-yn-1-ol to obtain the title compound (25 mg, yield: 54%) in a manner similar to Example 53.
¹H NMR (500 MHz, DMSO-d₆) δ 10.98 (s, 1H), 8.52 (d, 1H), 8.45 (s, 1H), 8.25 (t, 1H), 7.81 (m, 2H), 7.60 (d, 2H), 6.27 (brs, 2H), 5.94 (d, 1H), 5.52 (s, 1H), 1.39 (s, 6H); MS m/z : 577[M+H]

### Example 59. N-(4-((2-amino-3-chloropyridin-4-yl)oxy)-3,5-difluorophenyl)-1-(1-(3-methoxypropyl)piperi din-4-yl)-5-(trifluoromethyl)-1H-pyrazole-4-carboxamide

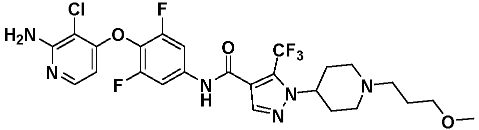

According to Example 1, 1-(1-(3-methoxypropyl)piperidin-4-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (6 mg, yield: 7%) in a manner similar to Example 1.
¹H NMR (500 MHz, DMSO-d₆) δ 7.95 (s, 1H), 7.77 (m, 2H), 7.60 (m, 2H), 6.05 (d, 1H), 4.60 (m, 1H), 5.52 (s, 1H), 2.85 (m, 2H), 2.75 (m, 2H), 2.42 (m, 2H), 2.15 (m, 2H), 1.95 (m, 2H); MS m/z : 590[M+H]

### Example 60. N-(4-((2-amino-3-chloropyridin-4-yl)oxy)-3,5-difluorophenyl)-1-(1-(2-ethoxyacetyl)piperi din-4-yl)-5-(trifluoromethyl)-1H-pyrazole-4-carboxamide

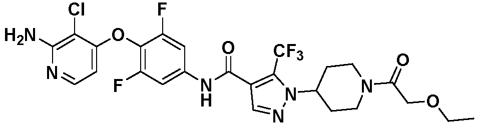

According to Example 1, 1-(1-(2-ethoxyacetyl)piperidin-4-yl)-5-(trifluoromethyl)-1H-pyrazole-4-carbonyl chloride was used instead of 1-phenyl-5-(trifluoromethyl)-1H-pyrazole-4-carbonyl chloride to obtain the title compound (12 mg, yield: 24%) in a manner similar to Example 1.
¹H NMR (500 MHz, DMSO-d₆) 10.93 (s, 1H), 8.10 (s, 1H), 7.61 (d, 2H), 6.50 (s, 2H), 6.02 (d, 1H), 4.67 (m, 1H), 4.54 (m, 2H), 4.06 (m, 2H), 3.62 (m, 2H), 3.45 (m, 3H), 3.24 (m, 2H), 2.96 (m, 1H), 2.85 (m, 1H), 2.79 (m, 1H), 2.05 (m, 6H); MS m/z : 603[M+H]

### Example 61. N-[4-[(2-amino-3-chloro-4-pyridyl)oxy]-3,5-difluoro-phenyl]-1-(3-methoxypropyl)-5-(trifluoromethyl)pyrazole-4-carboxamide

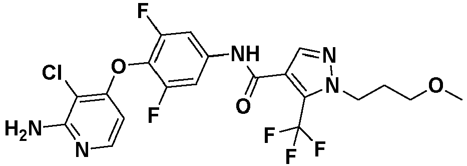

According to Example 1, 1-(3-methoxypropyl)-5-(trifluoromethyl)-1H-pyrazole-4-carbonyl chloride was used instead of 1-phenyl-5-(trifluoromethyl)-1H-pyrazole-4-carbonyl chloride to obtain the title compound (32 mg, yield: 44%) in a manner similar to Example 1.
¹H NMR (500 MHz, DMSO-d₆) δ 10.88 (s, mH), 8.59 (m, 1H), 7.78 (m, 2H), 7.66 (d, 2H), 6.50 (d, 2H), 6.03 (d, 1H), 4.37 (m, 2H), 3.36 (s, 3H), 3.25 (m, 2H), 2.08 (m, 2H); MS m/z : 506[M+H]

### Experimental Example 1. Evaluation of RON and MET inhibitory activity

### [1-1] Evaluation of RON inhibitory activity

To measure the RON inhibitory activity of the example compounds, the IC₅₀ of the compounds was measured using time-resolved fluorescence energy transfer (TR-FRET).

Specifically, the compound was prepared at a concentration of 1 mM with 100% DMSO and serially diluted 10 times through 3-fold dilution. 2 µl of the serially diluted compound was added to a 96-well plate containing 48 µl of 1x kinase reaction buffer (50 mM HEPES (pH 7.4), 0.01% Tween-20, 5 mM DTT, 0.5 mM Na₃VO₄, 2 mM EGTA, 10 mM MgCl₂).

The RON protein was diluted with a RON storage buffer (50 mM Tris-HCl (pH 7.5), 150 mM NaCl, 0.5 mM EDTA, 0.02% Triton X-100) to a concentration of 49.3 nM, and then diluted again with 1x kinase reaction buffer to be 0.4 nM. As a substrate cocktail, a RON-specific substrate mixture (40 µM ULight-labeled Poly GT, 100 nM ATP) was prepared at a concentration twice the final reaction concentration.

After that, a 384-well plate was prepared, and 2.5 µl of the diluted example compound was added to each of the experimental group wells, and 2.5 µl of a 4% DMSO solution was dispensed into high control wells and low control wells.

Afterward, 2.5 µl of 0.4 nM RON was dispensed into the high control wells and the experimental group wells, and 2.5 µl of 1x kinase reaction buffer was added to the low control wells, and after centrifugation at 1000 RPM for 40 seconds at room temperature, the mixtures were incubated at room temperature for 20 to 30 minutes. Then, 5 µl of the substrate cocktail (2x) was dispensed into all wells, and after centrifugation at 1000 RPM for 40 seconds at room temperature, the mixtures were incubated at room temperature for 60 minutes. Afterward, 5 µl of 30 mM EDTA was added to all wells to terminate the reaction, and the mixtures were incubated again at room temperature for 5 minutes. Thereafter, 5 µl of 4x phosphotyrosine antibodies (Perkin Elmer) containing europium was added to all wells and incubated at room temperature for 60 minutes. After the incubation, the 384-well-plate was read with a Vison plate reader. At this time, the excitation wavelength was 340 nm, and the emission wavelengths were 620 nm and 665 nm. The IC₅₀ value of each example compound was derived using the GraphPd Prism7 program.

### [1-2] Evaluation of MET inhibitory activity

In addition, to measure cMET enzyme activity inhibition, an experiment was performed by the same method as the experiment for measuring the RON enzyme activity inhibition.

Specifically, the compound was prepared at a concentration of 1 mM with 100% DMSO and serially diluted 10 times through 3-fold dilution. 2 µl of the serially diluted compound was added to a 96-well plate containing 48 µl 1x kinase reaction buffer (50 mM HEPES (pH 7.4), 0.05% BSA, 0.005% Tween-20, 1 mM DTT, 0.5 mM MnCl₂, 20 mM MgCl₂).

The cMET protein was diluted in a cMET storage buffer (50 mM Tris-HCl (pH 7.5), 150 mM NaCl, 0.05% Brij35, 1 mM DTT, 10% glycerol) to a concentration of 263 nM, and then diluted again with 1x kinase reaction buffer to 2 nM. As a substrate cocktail, a MET-specific substrate mixture (5 µM TK peptide, 10 mM ATP) was prepared at a concentration twice the final reaction concentration.

After that, a 384-well plate was prepared, and 2.5 µl of the diluted example compound was added to each of the experimental group wells, and 2.5 µl of a 4% DMSO solution was dispensed into high control wells and low control wells.

Afterward, 2.5 µl of 2 nM cMET was dispensed to the high control wells and the experimental group wells, and 2.5 µl of 1x kinase reaction buffer was added to the low control wells, and after centrifugation at 1000 RPM for 40 seconds at room temperature, the mixtures were incubated at room temperature for 20 to 30 minutes. Then, 5 µl of the substrate cocktail (2x) was dispensed into all wells, and after centrifugation at 1000 RPM for 40 seconds at room temperature, the mixtures were incubated at room temperature for 60 minutes. Afterward, 5 µl of 90 mM EDTA was added to all wells to terminate the reaction, and the mixtures were incubated again at room temperature for 5 minutes. Thereafter, 5 µl of 4x phosphotyrosine antibodies (Perkin Elmer) containing europium was added to all wells and incubated at room temperature for 60 minutes. After the incubation, the 384-well-plate was read with a Vison plate reader. At this time, the excitation wavelength was 340 nm, and the emission wavelengths were 620 nm and 665 nm. The IC₅₀ value of each example compound was derived using the GraphPd Prism7 program.

The results of the evaluation of the RON or cMET enzyme inhibitory activity of the example compounds are shown in Table 2 below.
(A: <50 nM, B: 50 to 500 nM, C: 500 to 5000 nM, D: >5,000 nM)

**[Table 2]**

| | RON (IC₅₀, nM) | MET (IC₅₀, nM) |
|---|---|---|
| Example 1 | A | D |
| Example 2 | A | C |
| Example 3 | A | C |
| Example 4 | A | D |
| Example 5 | A | D |
| Example 6 | B | D |
| Example 7 | B | D |
| Example 8 | C | D |
| Example 9 | A | D |
| Example 10 | A | C |
| Example 11 | A | C |
| Example 12 | A | C |
| Example 13 | A | C |
| Example 14 | A | D |
| Example 15 | A | B |
| Example 16 | A | B |
| Example 17 | A | C |
| Example 18 | A | C |
| Example 19 | A | C |
| Example 20 | A | B |
| Example 21 | A | B |
| Example 22 | A | C |
| Example 23 | A | C |
| Example 24 | A | C |
| Example 25 | A | C |
| Example 26 | A | C |
| Example 27 | A | B |
| Example 28 | A | C |
| Example 29 | A | C |
| Example 30 | A | C |
| Example 31 | A | C |
| Example 32 | A | C |
| Example 33 | A | B |
| Example 34 | A | B |
| Example 35 | A | C |
| Example 36 | B | C |
| Example 37 | A | C |
| Example 38 | A | C |
| Example 39 | A | C |
| Example 40 | A | C |
| Example 41 | A | C |
| Example 42 | A | C |
| Example 43 | A | C |
| Example 44 | A | C |
| Example 45 | A | C |
| Example 46 | A | C |
| Example 47 | A | C |
| Example 48 | A | D |
| Example 49 | A | C |
| Example 50 | A | C |
| Example 51 | A | C |
| Example 52 | A | B |
| Example 53 | A | B |
| Example 54 | A | B |
| Example 55 | A | B |
| Example 56 | A | B |
| Example 57 | A | B |
| Example 58 | A | B |
| Example 59 | C | D |
| Example 60 | C | C |
| Example 61 | B | C |
| A: <50 nM, B: 50 ~ 500 nM, C: 500 ~ 5000 nM, D: >5000 nM | | |

## Claims

1. A compound represented by Chemical Formula 1 below, or a pharmaceutically acceptable salt thereof: 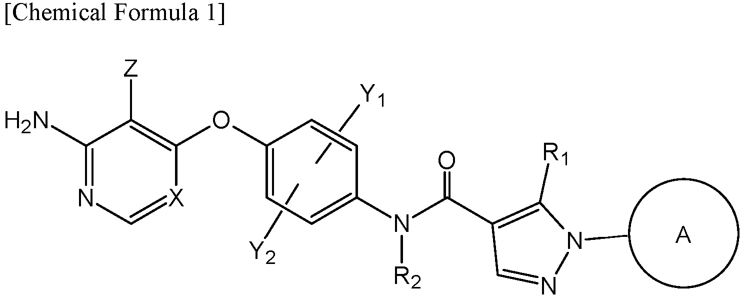 in Chemical Formula 1,
X is N or C;
R₁ is any one selected from the group consisting of hydrogen, a halogen, a substituted or unsubstituted alkyl, and a substituted or unsubstituted cycloalkyl;
R₂ is any one selected from the group consisting of hydrogen and a substituted or unsubstituted alkyl;
Y₁ and Y₂ are each independently any one selected from the group consisting of hydrogen, a halogen, a substituted or unsubstituted alkyl, and a substituted or unsubstituted cycloalkyl;
Z is any one selected from the group consisting of hydrogen, a halogen, a substituted or unsubstituted alkyl, a substituted or unsubstituted alkenyl, a substituted or unsubstituted alkynyl, a substituted or unsubstituted cycloalkyl, a substituted or unsubstituted heterocycloalkyl, a substituted or unsubstituted aryl, a substituted or unsubstituted heteroaryl, a nitro, a cyano, and -(CH₂)ₙ-W, wherein n is an integer from 0 to 3, W is -NRₐR_{b}, -ORₐ or a heterocycloalkyl, wherein Rₐ and R_{b} are each independently hydrogen or a substituted or unsubstituted alkyl;
A is any one selected from the group consisting of hydrogen, a substituted or unsubstituted alkyl, a substituted or unsubstituted cycloalkyl, a substituted or unsubstituted heterocycloalkyl, a substituted or unsubstituted aryl, and a substituted and unsubstituted heteroaryl; and
the substituted functional groups are groups substituted with at least one selected from the group consisting of a halogen, an alkyl, a hydroxyalkyl, a hydroxy, an alkoxy, an haloalkyl, an alkoxyalkyl, -(CO)-(CH₂)ₙ-OR_{c}, and an aryl, wherein n is an integer from 0 to 3, and R_{c} is hydrogen or an alkyl.

2. The compound or pharmaceutically acceptable salt thereof according to claim 1,
wherein R₁ is hydrogen, a halogen, an alkyl, or a haloalkyl;
R₂ is hydrogen or an alkoxyalkyl;
Y₁ and Y₂ are each independently hydrogen or a halogen;
Z is any one selected from the group consisting of hydrogen, a halogen, a substituted or unsubstituted alkyl, a substituted or unsubstituted alkynyl, a substituted or unsubstituted cycloalkyl, a substituted or unsubstituted heterocycloalkyl, a substituted or unsubstituted aryl, a nitro, a cyano, and -(CH₂)ₙ-W, wherein W is a heterocycloalkyl, and n is an integer from 1 to 3;
A is any one selected from the group consisting of a substituted or unsubstituted alkyl, a substituted or unsubstituted heterocycloalkyl, a substituted or unsubstituted aryl, and a substituted or unsubstituted heteroaryl; and
the substituted functional groups are groups substituted with at least one selected from the group consisting of a halogen, an alkyl, a hydroxyalkyl, a hydroxy, an alkoxy, an haloalkyl, an alkoxyalkyl, -(CO)-(CH₂)ₙ-OR_{c}, and an aryl, wherein n is an integer from 1 to 3, and R_{c} is hydrogen or an alkyl.

3. The compound or pharmaceutically acceptable salt thereof according to claim 1,
wherein R₁ is a halogen, an alkyl or a haloalkyl;
R₂ is hydrogen or an alkoxyalkyl;
Y₁ and Y₂ are each a halogen;
Z is any one selected from the group consisting of a halogen, a substituted or unsubstituted alkyl, a substituted or unsubstituted alkynyl, a substituted or unsubstituted cycloalkyl, a substituted or unsubstituted heterocycloalkyl, a cyano, and -(CH₂)ₙ-W, wherein n is an integer from 1 to 2 and W is a heterocycloalkyl;
A is any one selected from the group consisting of a substituted alkyl, a substituted heterocycloalkyl, a substituted or unsubstituted aryl, and a substituted or unsubstituted heteroaryl; and
the substituted functional groups are groups substituted with at least one selected from the group consisting of a halogen, an alkyl, a hydroxyalkyl, a hydroxy, an alkoxy, an haloalkyl, an alkoxyalkyl, and -(CO)-(CH₂)ₙ-OR_{c}, wherein n is an integer from 1 to 2, and R_{c} is hydrogen or an alkyl.

4. The compound or pharmaceutically acceptable salt thereof according to claim 1,
wherein R₁ is an alkyl or a haloalkyl;
R₂ is hydrogen or an alkoxyalkyl;
Y₁ and Y₂ are each fluorine;
Z is any one selected from the group consisting of a halogen, an alkyl, an alkynyl, a hydroxyalkynyl, a hydroxyalkylakynyl, a cycloalkyl, tetrahydropyran, a cyano, and -(CH₂)ₙ-W, wherein W is morpholine and n is 1; and
A is any one selected from the group consisting of an alkoxyalkyl, an aryl, a pyridine, a halopyridine, a pyrimidine, a pyridazine, piperidine, an alkoxyalkylpiperidine, and a piperidine group substituted with -(CO)-(CH₂)ₙ-OR_{c}, and where n is 1 and R_{c} is an alkyl.

5. The compound or pharmaceutically acceptable salt thereof according to claim 1, wherein the compound is selected from:
N-(4-((2-amino-3-chloropyridin-4-yl)oxy)-3,5-difluorophenyl)-1-phenyl-5-(trifluoromethyl)-1H-pyrazole-4-carboxamide;
N-(4-((2-amino-3-chloropyridin-4-yl)oxy)-3,5-difluorophenyl)-1-(pyrimidin-2-yl)-5-(trifluoromethyl)-1H-pyrazole-4-carboxamide;
N-(4-((2-amino-3-chloropyridin-4-yl)oxy)-3,5-difluorophenyl)-1-(pyridazin-3-yl)-5-(trifluoromethyl)-1H-pyrazole-4-carboxamide;
N-(4-((2-amino-3-chloropyridin-4-yl)oxy)-3,5-difluorophenyl)-5-ethyl-1-(3-fluoropyridin-2-yl)-1H-pyrazole-4-carboxamide;
N-(4-((2-amino-3-chloropyridin-4-yl)oxy)-2,5-difluorophenyl)-1-(3-fluoropyridin-2-yl)-5-(trifluoromethyl)-1H-pyrazole-4-carboxamide;
N-(4-((2-amino-3-chloropyridin-4-yl)oxy)-2,5-difluorophenyl)-1-phenyl-5-(trifluoromethyl)-1H-pyrazole-4-carboxamide;
N-(4-((2-amino-3-chloropyridin-4-yl)oxy)-2,6-difluorophenyl)-1-(3-fluoropyridin-2-yl)-5-(trifluoromethyl)-1H-pyrazole-4-carboxamide;
N-(4-((2-amino-3-chloropyridin-4-yl)oxy)-2,6-difluorophenyl)-1-phenyl-5-(trifluoromethyl)-1H-pyrazole-4-carboxamide;
N-(4-((6-amino-5-chloropyrimidin-4-yl)oxy)-3,5-difluorophenyl)-1-phenyl-5-(trifluoromethyl)-1H-pyrazole-4-carboxamide;
N-(4-((6-amino-5-chloropyrimidin-4-yl)oxy)-3,5-difluorophenyl)-1-(3-fluoropyridin-2-yl)-5-(trifluoromethyl)-1H-pyrazole-4-carboxamide;
N-(4-((6-amino-5-chloropyrimidin-4-yl)oxy)-3,5-difluorophenyl)-1-(3-chloropyridin-2-yl)-5-(trifluoromethyl)-1H-pyrazole-4-carboxamide;
N-(4-((6-amino-5-chloropyrimidin-4-yl)oxy)-3,5-difluorophenyl)-1-(pyrimidin-2-yl)-5-(trifluoromethyl)-1H-pyrazole-4-carboxamide;
N-(4-((2-amino-3-iodopyridin-4-yl)oxy)-3,5-difluorophenyl)-1-phenyl-5-(trifluoromethyl)-1H-pyrazole-4-carboxamide;
N-(4-((2-amino-3-iodopyridin-4-yl)oxy)-3,5-difluorophenyl)-1-(pyridin-2-yl)-5-(trifluoromethyl)-1H-pyrazole-4-carboxamide;
N-(4-((2-amino-3-iodopyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-iodopyridin-4-yl)oxy)-3,5-difluorophenyl)-1-(3-chloropyridin-2-yl)-5-(trifluoromethyl)-1H-pyrazole-4-carboxamide;
N-(4-((2-amino-3-iodopyridin-4-yl)oxy)-3,5-difluorophenyl)-1-(pyridazin-3-yl)-5-(trifluoromethyl)-1H-pyrazole-4-carboxamide;
N-(4-((2-amino-3-iodopyridin-4-yl)oxy)-3,5-difluorophenyl)-1-(pyrimidin-2-yl)-5-(trifluoromethyl)-1H-pyrazole-4-carboxamide;
N-(4-((2-amino-3-(tetrahydro-2H-pyran-4-yl)pyridin-4-yl)oxy)-3,5-difluorophenyl)-1-phenyl-5-(trifluoromethyl)-1H-pyrazole-4-carboxamide;
N-(4-((2-amino-3-(tetrahydro-2H-pyran-4-yl)pyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-(tetrahydro-2H-pyran-4-yl)pyridin-4-yl)oxy)-3,5-difluorophenyl)-1-(3-chloropyridin-2-yl)-5-(trifluoromethyl)-1H-pyrazole-4-carboxamide;
N-(4-((2-amino-3-(tetrahydro-2H-pyran-4-yl)pyridin-4-yl)oxy)-3,5-difluorophenyl)-1-(pyrimidin-2-yl)-5-(trifluoromethyl)-1H-pyrazole-4-carboxamide;
N-(4-((2-amino-3-(tetrahydro-2H-pyran-4-yl)pyridin-4-yl)oxy)-3,5-difluorophenyl)-1-(pyridazin-3-yl)-5-(trifluoromethyl)-1H-pyrazole-4-carboxamide;
N-(4-((2-amino-3-(tetrahydro-2H-pyran-4-yl)pyridin-4-yl)oxy)-3,5-difluorophenyl)-1-(pyridin-2-yl)-5-(trifluoromethyl)-1H-pyrazole-4-carboxamide;
N-(4-((2-amino-3-ethynylpyridin-4-yl)oxy)-3,5-difluorophenyl)-1-phenyl-5-(trifluoromethyl)-1H-pyrazole-4-carboxamide;
N-(4-((2-amino-3-ethynylpyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-ethynylpyridin-4-yl)oxy)-3,5-difluorophenyl)-1-(3-chloropyridin-2-yl)-5-(trifluoromethyl)-1H-pyrazole-4-carboxamide;
N-(4-((2-amino-3-ethynylpyridin-4-yl)oxy)-3,5-difluorophenyl)-1-(pyrimidin-2-yl)-5-(trifluoromethyl)-1H-pyrazole-4-carboxamide;
N-(4-((2-amino-3-ethynylpyridin-4-yl)oxy)-3,5-difluorophenyl)-1-(pyridin-2-yl)-5-(trifluoromethyl)-1H-pyrazole-4-carboxamide;
N-(4-((2-amino-3-ethynylpyridin-4-yl)oxy)-3,5-difluorophenyl)-1-(pyridazin-3-yl)-5-(trifluoromethyl)-1H-pyrazole-4-carboxamide;
N-(4-((2-amino-3-cyanopyridin-4-yl)oxy)-3,5-difluorophenyl)-1-phenyl-5-(trifluoromethyl)-1H-pyrazole-4-carboxamide;
N-(4-((2-amino-3-cyanopyridin-4-yl)oxy)-3,5-difluorophenyl)-1-(pyridin-2-yl)-5-(trifluoromethyl)-1H-pyrazole-4-carboxamide;
N-(4-((2-amino-3-cyanopyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-cyanopyridin-4-yl)oxy)-3,5-difluorophenyl)-1-(3-chloropyridin-2-yl)-5-(trifluoromethyl)-1H-pyrazole-4-carboxamide;
N-(4-((2-amino-3-cyanopyridin-4-yl)oxy)-3,5-difluorophenyl)-1-(pyrimidin-2-yl)-5-(trifluoromethyl)-1H-pyrazole-4-carboxamide;
N-(4-((2-amino-3-cyanopyridin-4-yl)oxy)-3,5-difluorophenyl)-1-(pyridazin-3-yl)-5-(trifluoromethyl)-1H-pyrazole-4-carboxamide;
N-(4-((2-amino-3-cyclopropylpyridin-4-yl)oxy)-3,5-difluorophenyl)-1-phenyl-5-(trifluoromethyl)-1H-pyrazole-4-carboxamide;
N-(4-((2-amino-3-cyclopropylpyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-cyclopropylpyridin-4-yl)oxy)-3,5-difluorophenyl)-1-(3-chloropyridin-2-yl)-5-(trifluoromethyl)-1H-pyrazole-4-carboxamide;
N-(4-((2-amino-3-cyclopropylpyridin-4-yl)oxy)-3,5-difluorophenyl)-1-(pyridin-2-yl)-5-(trifluoromethyl)-1H-pyrazole-4-carboxamide;
N-(4-((2-amino-3-cyclopropylpyridin-4-yl)oxy)-3,5-difluorophenyl)-1-(pyrimidin-2-yl)-5-(trifluoromethyl)-1H-pyrazole-4-carboxamide;
N-(4-((2-amino-3-cyclopropylpyridin-4-yl)oxy)-3,5-difluorophenyl)-1-(pyridazin-3-yl)-5-(trifluoromethyl)-1H-pyrazole-4-carboxamide;
N-(4-((2-amino-3-isopropylpyridin-4-yl)oxy)-3,5-difluorophenyl)-1-phenyl-5-(trifluoromethyl)-1H-pyrazole-4-carboxamide;
N-(4-((2-amino-3-isopropylpyridin-4-yl)oxy)-3,5-difluorophenyl)-1-(3-chloropyridin-2-yl)-5-(trifluoromethyl)-1H-pyrazole-4-carboxamide;
N-(4-((2-amino-3-isopropylpyridin-4-yl)oxy)-3,5-difluorophenyl)-1-(pyrimidin-2-yl)-5-(trifluoromethyl)-1H-pyrazole-4-carboxamide;
N-(4-((2-amino-3-isopropylpyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-(morpholinomethyl)pyridin-4-yl)oxy)-3,5-difluorophenyl)-1-(3-fluoropyridine-2-yl)-5-(trifluoromethyl)-1H-pyrazole-4-carboxamide;
N-(4-((2-amino-3-(morpholinomethyl)pyridin-4-yl)oxy)-3,5-difluorophenyl)-1-(pyrimidin-2-yl)-5-(trifluoromethyl)-1H-pyrazole-4-carboxamide;
N-(4-((2-amino-3-(morpholinomethyl)pyridin-4-yl)oxy)-3,5-difluorophenyl)-1-(3-chloropyridin-2-yl)-5-(trifluoromethyl)-1H-pyrazole-4-carboxamide;
N-(4-((2-amino-3-(morpholinomethyl)pyridin-4-yl)oxy)-3,5-difluorophenyl)-1-phenyl-5-(trifluoromethyl)-1H-pyrazole-4-carboxamide;
N-(4-((2-amino-3-(3-hydroxyprop-1-yn-1-yl)pyridin-4-yl)oxy)-3,5-difluorophenyl)-1-phenyl-5-(trifluoromethyl)-1H-pyrazole-4-carboxamide;
N-(4-((2-amino-3-(3-hydroxyprop-1-yn-1-yl)pyridin-4-yl)oxy)-3,5-difluorophenyl)-1-(pyridin-2-yl)-5-(trifluoromethyl)-1H-pyrazole-4-carboxamide;
N-(4-((2-amino-3-(3-hydroxyprop-1-yn-1-yl)pyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-(3-hydroxyprop-1-yn-1-yl)pyridin-4-yl)oxy)-3,5-difluorophenyl)-1-(3-chloropyridin-2-yl)-5-(trifluoromethyl)-1H-pyrazole-4-carboxamide;
N-(4-((2-amino-3-(3-hydroxyprop-1-yn-1-yl)pyridin-4-yl)oxy)-3,5-difluorophenyl)-1-(pyrimidin-2-yl)-5-(trifluoromethyl)-1H-pyrazole-4-carboxamide;
N-(4-((2-amino-3-(3-hydroxyprop-1-yn-1-yl)pyridin-4-yl)oxy)-3,5-difluorophenyl)-1-(pyridazin-3-yl)-5-(trifluoromethyl)-1H-pyrazole-4-carboxamide;
N-(4-((2-amino-3-(3-hydroxybut-1-yn-1-yl)pyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-(3-hydroxy-3-methylbut-1-yn-1-yl)pyridin-4-yl)oxy)-3,5-difluorophenyl)-1-(3-fluoropyridin-2-yl)-5-(trifluoromethyl)-1H-pyrazole-4-carboxamide;
N-(4-((2-amino-3-chloropyridin-4-yl)oxy)-3,5-difluorophenyl)-1-(1-(3-methoxypropyl)piperi din-4-yl)-5-(trifluoromethyl)-1H-pyrazole-4-carboxamide;
N-(4-((2-amino-3-chloropyridin-4-yl)oxy)-3,5-difluorophenyl)-1-(1-(2-ethoxyacetyl)piperi din-4-yl)-5-(trifluoromethyl)-1H-pyrazole-4-carboxamide; and
N-[4-[(2-amino-3-chloro-4-pyridyl)oxy]-3,5-difluoro-phenyl]-1-(3-methoxypropyl)-5-(trifluoromethyl)pyrazole-4-carboxamide.

6. A pharmaceutical composition comprising: the compound or pharmaceutically acceptable salt thereof according to any one of claims 1 to 5; and a pharmaceutically acceptable carrier.
